(19) **Europäisches Patentamt**
**European Patent Office**
**Office européen des brevets**

(11) **EP 4 286 412 A1**

(12) **EUROPEAN PATENT APPLICATION**
published in accordance with Art. 153(4) EPC

(43) Date of publication:
06.12.2023 Bulletin 2023/49

(21) Application number: 22746257.9

(22) Date of filing: 27.01.2022

(51) International Patent Classification (IPC):
$C07K\ 16/28^{(2006.01)}$  $A61K\ 47/68^{(2017.01)}$
$A61P\ 25/00^{(2006.01)}$  $A61P\ 25/28^{(2006.01)}$
$A61K\ 39/00^{(2006.01)}$

(52) Cooperative Patent Classification (CPC):
A61K 39/00; A61K 47/68; A61P 25/00;
A61P 25/28; C07K 16/18; C07K 16/28; G01N 33/68

(86) International application number:
PCT/KR2022/001498

(87) International publication number:
WO 2022/164231 (04.08.2022 Gazette 2022/31)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 27.01.2021 KR 20210011845

(71) Applicant: **Good T Cells, Inc.**
**Seoul 03929 (KR)**

(72) Inventors:
• **KIM, Jung Ho**
  **Seoul 04136 (KR)**
• **KIM, Beom Seok**
  **Seoul 03929 (KR)**

(74) Representative: **Pharma Patents International AG**
**Leonhardsgraben 52**
**Postfach**
**4001 Basel (CH)**

Remarks:
The complete document including Reference Table(s) and the Sequence Listing(s) can be downloaded from the EPO website

(54) **NOVEL BINDING MOLECULE AND USE THEREOF**

(57)    The present invention relates to a novel binding molecule with excellent preventive, ameliorative or therapeutic effects on a brain and nervous system disease and uses thereof. The binding molecule provided in the present invention can effectively prevent or treat brain and nervous system diseases, particularly neurodegenerative diseases or neuroinflammatory diseases.

【FIG. 12】

A: WT
B: 5xFAD Tg
C: +GA
D: GTC110-04
E: GTC310-01

EP 4 286 412 A1

Sequence List Free Text

<110>    Good T Cells, Inc.

<120>    Novel binding molecule and use thereof

<130>    POPB214473PCT

<160>    54

<170>    KoPatentIn 3.0

<210>    1

<211>    759

<212>    PRT

<213>    Homo sapiens

<400>    1

Gly Pro Arg Ala Pro Cys Ala Ala Ala Cys Thr Cys Ala Gly Asp Ser
1               5                   10                  15

Leu Asp Cys Gly Gly Arg Gly Leu Ala Ala Leu Pro Gly Asp Leu Pro

20              25              30

Ser Trp Thr Arg Ser Leu Asn Leu Ser Tyr Asn Lys Leu Ser Glu Ile

35              40              45

Asp Pro Ala Gly Phe Glu Asp Leu Pro Asn Leu Gln Glu Val Tyr Leu

50              55              60

Asn Asn Asn Glu Leu Thr Ala Val Pro Ser Leu Gly Ala Ala Ser Ser

65              70              75              80

His Val Val Ser Leu Phe Leu Gln His Asn Lys Ile Arg Ser Val Glu

85              90              95

Gly Ser Gln Leu Lys Ala Tyr Leu Ser Leu Glu Val Leu Asp Leu Ser

100                     105                     110

Leu Asn Asn Ile Thr Glu Val Arg Asn Thr Cys Phe Pro His Gly Pro

115                     120                     125

Pro Ile Lys Glu Leu Asn Leu Ala Gly Asn Arg Ile Gly Thr Leu Glu

130                     135                     140

Leu Gly Ala Phe Asp Gly Leu Ser Arg Ser Leu Leu Thr Leu Arg Leu

145                     150                     155                     160

Ser Lys Asn Arg Ile Thr Gln Leu Pro Val Arg Ala Phe Lys Leu Pro

165                     170                     175

Arg Leu Thr Gln Leu Asp Leu Asn Arg Asn Arg Ile Arg Leu Ile Glu

180     185     190

Gly Leu Thr Phe Gln Gly Leu Asn Ser Leu Glu Val Leu Lys Leu Gln

195     200     205

Arg Asn Asn Ile Ser Lys Leu Thr Asp Gly Ala Phe Trp Gly Leu Ser

210     215     220

Lys Met His Val Leu His Leu Glu Tyr Asn Ser Leu Val Glu Val Asn

225     230     235     240

Ser Gly Ser Leu Tyr Gly Leu Thr Ala Leu His Gln Leu His Leu Ser

245     250     255

Asn Asn Ser Ile Ala Arg Ile His Arg Lys Gly Trp Ser Phe Cys Gln

260                    265                    270

Lys Leu His Glu Leu Val Leu Ser Phe Asn Asn Leu Thr Arg Leu Asp

275                    280                    285

Glu Glu Ser Leu Ala Glu Leu Ser Ser Leu Ser Val Leu Arg Leu Ser

290                    295                    300

His Asn Ser Ile Ser His Ile Ala Glu Gly Ala Phe Lys Gly Leu Arg

305                    310                    315                    320

Ser Leu Arg Val Leu Asp Leu Asp His Asn Glu Ile Ser Gly Thr Ile

325                    330                    335

Glu Asp Thr Ser Gly Ala Phe Ser Gly Leu Asp Ser Leu Ser Lys Leu

340    345    350

Thr Leu Phe Gly Asn Lys Ile Lys Ser Val Ala Lys Arg Ala Phe Ser

355    360    365

Gly Leu Glu Gly Leu Glu His Leu Asn Leu Gly Gly Asn Ala Ile Arg

370    375    380

Ser Val Gln Phe Asp Ala Phe Val Lys Met Lys Asn Leu Lys Glu Leu

385    390    395    400

His Ile Ser Ser Asp Ser Phe Leu Cys Asp Cys Gln Leu Lys Trp Leu

405    410    415

Pro Pro Trp Leu Ile Gly Arg Met Leu Gln Ala Phe Val Thr Ala Thr

420                  425                 430

Cys Ala His Pro Glu Ser Leu Lys Gly Gln Ser Ile Phe Ser Val Pro

435                  440                 445

Pro Glu Ser Phe Val Cys Asp Asp Phe Leu Lys Pro Gln Ile Ile Thr

450                  455                 460

Gln Pro Glu Thr Thr Met Ala Met Val Gly Lys Asp Ile Arg Phe Thr

465                  470                475                480

Cys Ser Ala Ala Ser Ser Ser Ser Ser Pro Met Thr Phe Ala Trp Lys

485                  490                495

Lys Asp Asn Glu Val Leu Thr Asn Ala Asp Met Glu Asn Phe Val His

500 505 510

Val His Ala Gln Asp Gly Glu Val Met Glu Tyr Thr Thr Ile Leu His

515 520 525

Leu Arg Gln Val Thr Phe Gly His Glu Gly Arg Tyr Gln Cys Val Ile

530 535 540

Thr Asn His Phe Gly Ser Thr Tyr Ser His Lys Ala Arg Leu Thr Val

545 550 555 560

Asn Val Leu Pro Ser Phe Thr Lys Thr Pro His Asp Ile Thr Ile Arg

565 570 575

Thr Thr Thr Val Ala Arg Leu Glu Cys Ala Ala Thr Gly His Pro Asn

580                          585                          590

Pro Gln Ile Ala Trp Gln Lys Asp Gly Gly Thr Asp Phe Pro Ala Ala

595                          600                          605

Arg Glu Arg Arg Met His Val Met Pro Asp Asp Asp Val Phe Phe Ile

610                          615                          620

Thr Asp Val Lys Ile Asp Asp Ala Gly Val Tyr Ser Cys Thr Ala Gln

625                          630                          635                          640

Asn Ser Ala Gly Ser Ile Ser Ala Asn Ala Thr Leu Thr Val Leu Glu

645                          650                          655

Thr Pro Ser Leu Val Val Pro Leu Glu Asp Arg Val Val Ser Val Gly

660 665 670

Glu Thr Val Ala Leu Gln Cys Lys Ala Thr Gly Asn Pro Pro Pro Arg

675 680 685

Ile Thr Trp Phe Lys Gly Asp Arg Pro Leu Ser Leu Thr Glu Arg His

690 695 700

His Leu Thr Pro Asp Asn Gln Leu Leu Val Val Gln Asn Val Val Ala

705 710 715 720

Glu Asp Ala Gly Arg Tyr Thr Cys Glu Met Ser Asn Thr Leu Gly Thr

725 730 735

Glu Arg Ala His Ser Gln Leu Ser Val Leu Pro Ala Ala Gly Cys Arg

740          745          750

Lys Asp Gly Thr Thr Val Gly

755

<210>    2

<211>    2397

<212>    DNA

<213>    Homo sapiens

<400>    2

ggcccgcggg cgccctgcgc ggccgcctgc acttgcgctg gggactcgct ggactgcggt

60

gggcgcgggc tggctgcgtt gcccggggac ctgccctcct ggacgcggag cctaaacctg

120

agttacaaca aactctctga gattgaccct gctggttttg aggacttgcc gaacctacag

180

gaagtgtacc tcaataataa tgagttgaca gcggtaccat ccctgggcgc tgcttcatca

240

catgtcgtct ctctctttct gcagcacaac aagattcgca gcgtggaggg gagccagctg

300

aaggcctacc tttccttaga agtgttagat ctgagtttga acaacatcac ggaagtgcgg

360

aacacctgct ttccacacgg accgcctata aaggagctca acctggcagg caatcggatt

420

ggcaccctgg agttgggagc atttgatggt ctgtcacggt cgctgctaac tcttcgcctg

480

agcaaaaaca ggatcaccca gcttcctgta agagcattca agctacccag gctgacacaa

540

ctggacctca atcggaacag gattcggctg atagagggcc tcaccttcca ggggctcaac

600

agcttggagg tgctgaagct tcagcgaaac aacatcagca actgacaga tggggccttc

660

tggggactgt ccaagatgca tgtgctgcac ctggagtaca acagcctggt agaagtgaac

720

agcggctcgc tctacggcct cacggccctg catcagctcc acctcagcaa caattccatc

780

gctcgcattc accgcaaggg ctggagcttc tgccagaagc tgcatgagtt ggtcctgtcc

840

ttcaacaacc tgacacggct ggacgaggag agcctggccg agctgagcag cctgagtgtc

900

ctgcgtctca gccacaattc catcagccac attgcggagg gtgccttcaa gggactcagg

960

agcctgcgag tcttggatct ggaccataac gagatttcgg gcacaataga ggacacgagc

1020

ggcgccttct cagggctcga cagcctcagc aagctgactc tgtttggaaa caagatcaag

1080

tctgtggcta agagagcatt ctcggggctg gaaggcctgg agcacctgaa ccttggaggg

1140

aatgcgatca gatctgtcca gtttgatgcc tttgtgaaga tgaagaatct taaagagctc

1200

catatcagca gcgacagctt cctgtgtgac tgccagctga gtggctgcc cccgtggcta

1260

attggcagga tgctgcaggc ctttgtgaca gccacctgtg cccacccaga atcactgaag

1320

ggtcagagca ttttctctgt gccaccagag agtttcgtgt gcgatgactt cctgaagcca

1380

cagatcatca cccagccaga aaccaccatg gctatggtgg gcaaggacat ccggtttaca

1440

tgctcagcag ccagcagcag cagctccccc atgacctttg cctggaagaa agacaatgaa

1500

gtcctgacca atgcagacat ggagaacttt gtccacgtcc acgcgcagga cggggaagtg

1560

atggagtaca ccaccatcct gcacctccgt caggtcactt tcgggcacga gggccgctac

1620

caatgtgtca tcaccaacca ctttggctcc acctattcac ataaggccag gctcaccgtg

1680

aatgtgttgc catcattcac caaaacgccc cacgacataa ccatccggac caccaccgtg

1740

gcccgcctcg aatgtgctgc cacaggtcac ccaaaccctc agattgcctg gcagaaggat

1800

ggaggcacgg atttccccgc tgcccgtgag cgacgcatgc atgtcatgcc ggatgacgac

1860

gtgttttttca tcactgatgt gaaaatagat gacgcagggg tttacagctg tactgctcag

1920

aactcagccg gttctatttc agctaatgcc accctgactg tcctagagac cccatccttg

1980

gtggtcccct tggaagaccg tgtggtatct gtgggagaaa cagtggccct ccaatgcaaa

2040

gccacgggga accctccgcc ccgcatcacc tggttcaagg gggaccgccc gctgagcctc

2100

actgagcggc accacctgac ccctgacaac cagctcctgg tggttcagaa cgtggtggca

2160

gaggatgcgg gccgatatac ctgtgagatg tccaacaccc tgggcacgga gcgagctcac

2220

agccagctga gcgtcctgcc cgcagcaggc tgcaggaagg atgggaccac ggtaggcatc

2280

ttcaccattg ctgtcgtgag cagcatcgtc ctgacgtcac tggtctgggt gtgcatcatc

2340

taccagacca ggaagaagag tgaagagtac agtgtcacca acacagatga aaccgtc

2397

<210>    3

<211> 761

<212> PRT

<213> Mus musculus

<400> 3

Gln Ala Gly Pro Arg Ala Pro Cys Ala Ala Ala Cys Thr Cys Ala Gly
1               5                   10                  15

Asp Ser Leu Asp Cys Ser Gly Arg Gly Leu Ala Thr Leu Pro Arg Asp
                20                  25                  30

Leu Pro Ser Trp Thr Arg Ser Leu Asn Leu Ser Tyr Asn Arg Leu Ser
                35                  40                  45

Glu Ile Asp Ser Ala Ala Phe Glu Asp Leu Thr Asn Leu Gln Glu Val

50                          55                          60

Tyr Leu Asn Ser Asn Glu Leu Thr Ala Ile Pro Ser Leu Gly Ala Ala

65                          70                          75                          80

Ser Ile Gly Val Val Ser Leu Phe Leu Gln His Asn Lys Ile Leu Ser

85                          90                          95

Val Asp Gly Ser Gln Leu Lys Ser Tyr Leu Ser Leu Glu Val Leu Asp

100                         105                         110

Leu Ser Ser Asn Asn Ile Thr Glu Ile Arg Ser Ser Cys Phe Pro Asn

115                         120                         125

Gly Leu Arg Ile Arg Glu Leu Asn Leu Ala Ser Asn Arg Ile Ser Ile

130          135          140

Leu Glu Ser Gly Ala Phe Asp Gly Leu Ser Arg Ser Leu Leu Thr Leu

145          150          155          160

Arg Leu Ser Lys Asn Arg Ile Thr Gln Leu Pro Val Lys Ala Phe Lys

165          170          175

Leu Pro Arg Leu Thr Gln Leu Asp Leu Asn Arg Asn Arg Ile Arg Leu

180          185          190

Ile Glu Gly Leu Thr Phe Gln Gly Leu Asp Ser Leu Glu Val Leu Arg

195          200          205

Leu Gln Arg Asn Asn Ile Ser Arg Leu Thr Asp Gly Ala Phe Trp Gly

210            215            220

Leu Ser Lys Met His Val Leu His Leu Glu Tyr Asn Ser Leu Val Glu

225            230            235            240

Val Asn Ser Gly Ser Leu Tyr Gly Leu Thr Ala Leu His Gln Leu His

245            250            255

Leu Ser Asn Asn Ser Ile Ser Arg Ile Gln Arg Asp Gly Trp Ser Phe

260            265            270

Cys Gln Lys Leu His Glu Leu Ile Leu Ser Phe Asn Asn Leu Thr Arg

275            280            285

Leu Asp Glu Glu Ser Leu Ala Glu Leu Ser Ser Leu Ser Ile Leu Arg

290                    295                    300

Leu Ser His Asn Ala Ile Ser His Ile Ala Glu Gly Ala Phe Lys Gly

305                    310                    315                    320

Leu Lys Ser Leu Arg Val Leu Asp Leu Asp His Asn Glu Ile Ser Gly

325                    330                    335

Thr Ile Glu Asp Thr Ser Gly Ala Phe Thr Gly Leu Asp Asn Leu Ser

340                    345                    350

Lys Leu Thr Leu Phe Gly Asn Lys Ile Lys Ser Val Ala Lys Arg Ala

355                    360                    365

Phe Ser Gly Leu Glu Ser Leu Glu His Leu Asn Leu Gly Glu Asn Ala

370                 375                 380

Ile Arg Ser Val Gln Phe Asp Ala Phe Ala Lys Met Lys Asn Leu Lys

385             390             395             400

Glu Leu Tyr Ile Ser Ser Glu Ser Phe Leu Cys Asp Cys Gln Leu Lys

405             410             415

Trp Leu Pro Pro Trp Leu Met Gly Arg Met Leu Gln Ala Phe Val Thr

420             425             430

Ala Thr Cys Ala His Pro Glu Ser Leu Lys Gly Gln Ser Ile Phe Ser

435             440             445

Val Leu Pro Asp Ser Phe Val Cys Asp Asp Phe Pro Lys Pro Gln Ile

450                    455                         460

Ile Thr Gln Pro Glu Thr Thr Met Ala Val Val Gly Lys Asp Ile Arg

465                    470                    475                    480

Phe Thr Cys Ser Ala Ala Ser Ser Ser Ser Ser Pro Met Thr Phe Ala

                  485                    490                    495

Trp Lys Lys Asp Asn Glu Val Leu Ala Asn Ala Asp Met Glu Asn Phe

                  500                    505                    510

Ala His Val Arg Ala Gln Asp Gly Glu Val Met Glu Tyr Thr Thr Ile

                  515                    520                    525

Leu His Leu Arg His Val Thr Phe Gly His Glu Gly Arg Tyr Gln Cys

530                    535                    540

Ile Ile Thr Asn His Phe Gly Ser Thr Tyr Ser His Lys Ala Arg Leu

545                    550                    555                    560

Thr Val Asn Val Leu Pro Ser Phe Thr Lys Ile Pro His Asp Ile Ala

565                    570                    575

Ile Arg Thr Gly Thr Thr Ala Arg Leu Glu Cys Ala Ala Thr Gly His

580                    585                    590

Pro Asn Pro Gln Ile Ala Trp Gln Lys Asp Gly Gly Thr Asp Phe Pro

595                    600                    605

Ala Ala Arg Glu Arg Arg Met His Val Met Pro Asp Asp Asp Val Phe

610                615                620

Phe Ile Thr Asp Val Lys Ile Asp Asp Met Gly Val Tyr Ser Cys Thr

625                630                635                640

Ala Gln Asn Ser Ala Gly Ser Val Ser Ala Asn Ala Thr Leu Thr Val

645                650                655

Leu Glu Thr Pro Ser Leu Ala Val Pro Leu Glu Asp Arg Val Val Thr

660                665                670

Val Gly Glu Thr Val Ala Phe Gln Cys Lys Ala Thr Gly Ser Pro Thr

675                680                685

Pro Arg Ile Thr Trp Leu Lys Gly Gly Arg Pro Leu Ser Leu Thr Glu

690                          695                          700

Arg His His Phe Thr Pro Gly Asn Gln Leu Leu Val Val Gln Asn Val

705                     710                     715                          720

Met Ile Asp Asp Ala Gly Arg Tyr Thr Cys Glu Met Ser Asn Pro Leu

725                     730                     735

Gly Thr Glu Arg Ala His Ser Gln Leu Ser Ile Leu Pro Thr Pro Gly

740                     745                     750

Cys Arg Lys Asp Gly Thr Thr Val Gly

755                     760

<210> 4

<211> 2283

<212> DNA

<213> Mus musculus

<400> 4

caggctggcc cgcgggcccc ctgcgcggcc gcctgcactt gcgccgggga ctcgctggac

60

tgcagtgggc gcgggctggc gacgctgccc cgggacctgc cctcctggac gcgcagccta

120

aacctgagtt ataacagact ctccgagatc gactctgctg cttttgagga cttgacgaat

180

ctgcaggaag tgtacctcaa cagcaatgag ctgacagcca taccatcact gggcgctgct

240

tccataggag ttgtctctct ctttttgcag cacaacaaga tccttagtgt ggatgggagc

300

cagctgaagt cgtacctgtc cttggaagtg ctggatctga gttccaacaa catcacggaa

360

attcggagct cctgtttccc gaacggcctg cgtataaggg aactcaactt ggcgagcaac

420

cgcatcagca tcctggagtc tggagcattt gatggtctgt cgcggtcact gctgactctc

480

cgtctgagca aaaacaggat cacccagctt cctgtgaaag cgttcaagct acccaggctg

540

acacaactag acctgaatcg gaatcggatt cggctgattg aaggcctcac gttccagggg

600

ctcgacagct tagaggtgct gaggcttcag aggaacaaca tcagcaggct gacggacggg

660

gccttctggg ggctgtctaa gatgcacgtg ctgcacctgg agtacaacag tctggtggaa

720

gtgaacagtg gctccctcta tggcctcaca gccctgcacc agctgcacct cagcaacaac

780

tccatctctc gaattcagcg tgatggctgg agcttctgcc aaaagctgca tgagttgatt

840

ctgtccttca acaacctcac gcggctggat gaggagagtc tagcggagtt gagcagcctc

900

agtatcctgc gcctcagtca caacgccatc agtcacattg ctgaaggcgc cttcaaggga

960

ctcaagagtc tgcgggtctt ggacctggac cataacgaga tctcgggtac aatcgaggat

1020

accagtggtg cctttacggg gcttgacaac ctcagcaagc tgactctgtt tggaaacaag

1080

atcaaatctg tggctaagag agccttctcg ggcctggaaa gcctggaaca cctgaacctt

ggagagaatg caatcaggtc tgtccagttt gatgcctttg caaagatgaa gaaccttaaa

gagctctaca tcagcagtga gagcttcctg tgtgactgcc agctcaagtg gctgccccca

tggctaatgg gtaggatgct gcaggccttt gtgacagcca cctgtgccca tccagagtcg

ctgaagggcc agagcatttt ctcagtgctg ccagacagct ttgtgtgtga tgactttcca

aagccacaga tcatcaccca gcctgagacg accatggctg tggtgggcaa ggacatccgt

1440

ttcacatgct ccgcagccag cagcagcagc tcaccaatga ccttcgcctg gaagaaggac

1500

aatgaggtcc tggccaatgc agacatggag aactttgccc acgtccgtgc acaggacggc

1560

gaagtgatgg agtataccac tatcctgcac ctccgtcacg tcacctttgg gcacgagggc

1620

cgctaccagt gtatcatcac aaaccacttt ggctccacat actcccacaa agccaggctc

1680

actgtgaatg tgttgccatc attcactaaa atacccatg acattgccat ccggactggc

1740

accacagccc gcctcgagtg tgctgccacg ggccacccta accctcagat tgcctggcag

1800

aaggatggag gcaccgattt cccggcagct cgtgagcgac gcatgcatgt tatgccagac

1860

gatgatgtgt tcttcatcac tgatgtgaaa atagacgaca tgggggtcta cagctgcact

1920

gcccagaact cggcaggctc ggtttcagcc aacgctaccc tcacagtctt agaaactcca

1980

tccttggcag tgcctctgga agaccgtgtg gtaactgtgg gagaaacagt ggccttccag

2040

tgcaaagcaa ccgggagccc cacaccacgc atcacctggc ttaagggagg tcgcccattg

2100

agcctcacag agcgccacca tttcactcca ggcaaccagc tgctggttgt tcagaatgtg

2160

atgatagacg atgcagggcg gtatacctgt gagatgtcta atcccctggg cactgagcga

2220

gcacatagcc agctgagcat tttacctacc cctggctgcc ggaaggatgg gaccaccgta

2280

ggc

2283

<210>    5

<211>    5

<212>    PRT

<213>    Artificial Sequence

<220>

<223>    Heavy chain CDR 1

<400>    5

Asn Tyr Ala Met Ser

1                    5

<210>    6

<211>    17

<212>    PRT

<213>    Artificial Sequence

<220>

<223>    Heavy chain CDR 2

<400>    6

Val Ile Ser His Gly Gly Gly Ser Thr Tyr Tyr Ala Asp Ser Val Lys

1                    5                    10                    15

Gly

<210>    7

<211>    18

<212>    PRT

<213>    Artificial Sequence

<220>

<223>    Heavy chain CDR 3

<400>    7

Val Ile Ser Asn Cys His Leu Gly Val Cys Tyr Tyr Ser Asn Gly Met

1                 5                 10                 15

Asp Val

<210>    8

<211>    13

<212>    PRT

<213>    Artificial Sequence

<220>

<223>    Light chain CDR 1

<400> 8

Ser Gly Ser Ser Ser Asn Ile Gly Asp Asn Tyr Val Ser

1                       5                       10

<210> 9

<211> 7

<212> PRT

<213> Artificial Sequence

<220>

*884<223> Light chain CDR 2

<400> 9

Asp Asn Asn Lys Arg Pro Ser

1               5

<210> 10

<211> 10

<212> PRT

<213> Artificial Sequence

<220>

<223> Light chain CDR 3

<400>    10

Trp Asp Gly Ser Leu Ser Ala Gly Arg Val

1                    5                    10

<210>    11

<211>    127

<212>    PRT

<213>    Artificial Sequence

<220>

<223>    Heavy chain variable region

<400>    11

Glu Val Gln Leu Leu Glu Ser Gly Gly Gly Leu Val Gln Pro Gly Gly

1 5 10 15

*918

Ser Leu Arg Leu Ser Cys Ala Ala Ser Gly Phe Thr Phe Ser Asn Tyr

20 25 30

Ala Met Ser Trp Val Arg Gln Ala Pro Gly Lys Gly Leu Glu Trp Val

35 40 45

Ser Val Ile Ser His Gly Gly Gly Ser Thr Tyr Tyr Ala Asp Ser Val

50 55 60

Lys Gly Arg Phe Thr Ile Ser Arg Asp Asn Ser Lys Asn Thr Leu Tyr

65 70 75 80

*930

Leu Gln Met Asn Ser Leu Arg Ala Glu Asp Thr Ala Val Tyr Tyr Cys

85                    90                    95

Ala Arg Val Ile Ser Asn Cys His Leu Gly Val Cys Tyr Tyr Ser Asn

100                   105                   110

Gly Met Asp Val Trp Gly Gln Gly Thr Leu Val Thr Val Ser Ser

115                   120                   125

<210>     12

<211>　111

<212>　PRT

<213>　Artificial Sequence

<220>

<223>　Light chain variable region

<400>　12

Gln Leu Val Leu Thr Gln Pro Pro Ser Val Ser Ala Ala Pro Gly Gln

1　　　　　　　　　5　　　　　　　　　10　　　　　　　　　15

*955

Arg Val Ser Ile Ser Cys Ser Gly Ser Ser Ser Asn Ile Gly Asp Asn

20　　　　　　　　　25　　　　　　　　　30

Tyr Val Ser Trp Tyr Gln Gln Val Pro Gly Ser Ala Pro Lys Leu Leu

35                    40                    45

Ile Tyr Asp Asn Asn Lys Arg Pro Ser Gly Ile Pro Asp Arg Phe Ser

50                    55                    60

Ala Ser Lys Ser Gly Thr Ser Ala Thr Leu Gly Ile Thr Gly Leu Gln

65                    70                    75                    80

*967

Thr Gly Asp Glu Ala Asp Tyr Tyr Cys Ala Thr Trp Asp Gly Ser Leu

85                    90                    95

Ser Ala Gly Arg Val Phe Gly Gly Gly Thr Lys Leu Thr Val Leu

100                    105                    110

<210>    13

<211>    15

<212>    DNA

<213>    Artificial Sequence

<220>

<223>    Heavy chain CDR 1

<400>    13

aactacgcca tgagc

15

<210>    14

<211>    51

<212>    DNA

<213>    Artificial Sequence

<220>

<223>    Heavy chain CDR 2

<400>    14

gtgatctctc acggcggagg cagcacctac tacgccgatt ctgtgaaggg c

51

<210> 15

<211> 54

<212> DNA

<213> Artificial Sequence

<220>

<223> Heavy chain CDR 3

*1009

<400> 15

gtgatcagca actgccacct gggcgtgtgc tactacagca acggcatgga tgtg

54

<210>     16

<211>     39

<212>     DNA

<213>     Artificial Sequence

<220>

<223>     Light chain CDR 1

*1022

<400>     16

agcggcagca gcagcaacat cggcgacaac tacgtgtcc

39

<210> 17

<211> 21

<212> DNA

<213> Artificial Sequence

<220>

<223> Light chain CDR 2

*1035

<400> 17

gacaacaaca agcggcccag c

21

<210>    18

<211>    30

<212>    DNA

<213>    Artificial Sequence

<220>

<223>    Light chain CDR 3

*1048

<400>    18

tgggatggct ccctgagcgc cggaagagtt                    30

<210> 19

<211> 381

<212> DNA

<213> Artificial Sequence

<220>

<223> Heavy chain variable region

<400> 19

gaagttcagc tgcttgagtc tggcggagga ctggttcaac ctggcggaag cctgagactg

60

tcttgtgccg ccagcggctt caccttcagc aactacgcca tgagctgggt ccgacaggcc

120

cctggaaaag gccttgaatg ggtgtccgtg atctctcacg gcggaggcag cacctactac

180

gccgattctg tgaagggcag attcaccatc agccgggaca acagcaagaa caccctgtac

240

ctgcagatga acagcctgag agccgaggac accgccgtgt actattgcgc cagagtgatc

300

agcaactgcc acctgggcgt gtgctactac agcaacggca tggatgtgtg gggccagggc

360

acactggtta ccgttagttc t

381

<210>    20

<211>    333

<212>    DNA

<213>    Artificial Sequence

<220>

<223>    Light chain variable region

<400>    20

caactggttc tgacacagcc tccaagcgtg tcagctgccc ctggacagag agtgtccatc

agctgtagcg gcagcagcag caacatcggc gacaactacg tgtcctggta tcagcaggtc

ccaggctctg cccctaagct gctgatctac gacaacaaca agcggcccag cggcatcccc

gatagatttt ctgccagcaa gagcggcacc agcgccacac tgggaattac aggactgcag

acaggcgacg aggccgacta ctattgtgcc acatgggatg gctccctgag cgccggaaga

gttttttggcg gaggcaccaa gctgaccgtg ctt

333

<210> 21

<211> 328

<212> PRT

<213> Mus musculus

<400> 21

Thr Thr Ala Pro Ser Val Tyr Pro Leu Ala Pro Val Cys Gly Asp Thr
1               5                   10                  15

Thr Gly Ser Ser Val Thr Leu Gly Cys Leu Val Lys Gly Tyr Phe Pro
                20                  25                  30

Glu Pro Val Thr Leu Thr Trp Asn Ser Gly Ser Leu Ser Ser Gly Val

35                    40                    45

His Thr Phe Pro Ala Val Leu Gln Ser Asp Leu Tyr Thr Leu Ser Ser

50                    55                    60

Ser Val Thr Val Thr Ser Ser Thr Trp Pro Ser Gln Ser Ile Thr Cys

65                    70                    75                    80

Asn Val Ala His Pro Ala Ser Ser Thr Lys Val Asp Lys Lys Ile Glu

85                    90                    95

Pro Arg Gly Pro Thr Ile Lys Pro Cys Pro Pro Cys Lys Cys Pro Ala

100                    105                    110

Pro Asn Leu Leu Gly Gly Pro Ser Val Phe Ile Phe Pro Pro Lys Ile

115                    120                    125

Lys Asp Val Leu Met Ile Ser Leu Ser Pro Ile Val Thr Cys Val Val

130                    135                    140

Val Asp Val Ser Glu Asp Asp Pro Asp Val Gln Ile Ser Trp Phe Val

145                    150                    155                    160

Asn Asn Val Glu Val His Thr Ala Gln Thr Gln Thr His Arg Glu Asp

165                    170                    175

Tyr Asn Ser Thr Leu Arg Val Val Ser Ala Leu Pro Ile Gln His Gln

180                    185                    190

Asp Trp Met Ser Gly Lys Glu Phe Lys Cys Lys Val Asn Asn Lys Asp

195 200 205

Leu Pro Ala Pro Ile Glu Arg Thr Ile Ser Lys Pro Lys Gly Ser Val

210 215 220

Arg Ala Pro Gln Val Tyr Val Leu Pro Pro Pro Glu Glu Glu Met Thr

225 230 235 240

Lys Lys Gln Val Thr Leu Thr Cys Met Val Thr Asp Phe Met Pro Glu

245 250 255

Asp Ile Tyr Val Glu Trp Thr Asn Asn Gly Lys Thr Glu Leu Asn Tyr

260 265 270

Lys Asn Thr Glu Pro Val Leu Asp Ser Asp Gly Ser Tyr Phe Met Tyr

275 280 285

Ser Lys Leu Arg Val Glu Lys Lys Asn Trp Val Glu Arg Asn Ser Tyr

290 295 300

Ser Cys Ser Val Val His Glu Gly Leu His Asn His His Thr Thr Lys

305 310 315 320

Ser Phe Ser Arg Thr Pro Gly Lys

325

<210> 22

<211> 107

<212>    PRT

<213>    Mus musculus

<400>    22

Arg Thr Val Ala Ala Pro Thr Val Ser Ile Phe Pro Pro Ser Ser Glu

1                5                10                15

Gln Leu Thr Ser Gly Gly Ala Ser Val Val Cys Phe Leu Asn Asn Phe

20                25                30

Tyr Pro Lys Asp Ile Asn Val Lys Trp Lys Ile Asp Gly Ser Glu Arg

35                40                45

Gln Asn Gly Val Leu Asn Ser Trp Thr Asp Gln Asp Ser Lys Asp Ser

50                55                60

Thr Tyr Ser Met Ser Ser Thr Leu Thr Leu Thr Lys Asp Glu Tyr Glu

65                    70                    75                    80

Arg His Asn Ser Tyr Thr Cys Glu Ala Thr His Lys Thr Ser Thr Ser

                          85                    90                    95

*1195

Pro Ile Val Lys Ser Phe Asn Arg Asn Glu Cys

                    100                    105

<210>     23

<211>     330

<212>     PRT

<213>     Homo sapiens

<400>     23

Ala Ser Thr Lys Gly Pro Ser Val Phe Pro Leu Ala Pro Ser Ser Lys

1                   5                   10                  15

Ser Thr Ser Gly Gly Thr Ala Ala Leu Gly Cys Leu Val Lys Asp Tyr

            20                  25                  30

Phe Pro Glu Pro Val Thr Val Ser Trp Asn Ser Gly Ala Leu Thr Ser

            35                  40                  45

Gly Val His Thr Phe Pro Ala Val Leu Gln Ser Ser Gly Leu Tyr Ser

            50                  55                  60

Leu Ser Ser Val Val Thr Val Pro Ser Ser Ser Leu Gly Thr Gln Thr

65 70 75 80

Tyr Ile Cys Asn Val Asn His Lys Pro Ser Asn Thr Lys Val Asp Lys

85 90 95

Lys Val Glu Pro Lys Ser Cys Asp Lys Thr His Thr Cys Pro Pro Cys

100 105 110

Pro Ala Pro Glu Leu Leu Gly Gly Pro Ser Val Phe Leu Phe Pro Pro

115 120 125

Lys Pro Lys Asp Thr Leu Met Ile Ser Arg Thr Pro Glu Val Thr Cys

130 135 140

Val Val Val Asp Val Ser His Glu Asp Pro Glu Val Lys Phe Asn Trp

145                  150                  155                  160

Tyr Val Asp Gly Val Glu Val His Asn Ala Lys Thr Lys Pro Arg Glu

165                  170                  175

Glu Gln Tyr Asn Ser Thr Tyr Arg Val Val Ser Val Leu Thr Val Leu

180                  185                  190

His Gln Asp Trp Leu Asn Gly Lys Glu Tyr Lys Cys Lys Val Ser Asn

195                  200                  205

Lys Ala Leu Pro Ala Pro Ile Glu Lys Thr Ile Ser Lys Ala Lys Gly

210                  215                  220

Gln Pro Arg Glu Pro Gln Val Tyr Thr Leu Pro Pro Ser Arg Asp Glu

225                    230                    235                    240

Leu Thr Lys Asn Gln Val Ser Leu Thr Cys Leu Val Lys Gly Phe Tyr

                    245                    250                    255

Pro Ser Asp Ile Ala Val Glu Trp Glu Ser Asn Gly Gln Pro Glu Asn

                    260                    265                    270

Asn Tyr Lys Thr Thr Pro Pro Val Leu Asp Ser Asp Gly Ser Phe Phe

                    275                    280                    285

Leu Tyr Ser Lys Leu Thr Val Asp Lys Ser Arg Trp Gln Gln Gly Asn

                    290                    295                    300

Val Phe Ser Cys Ser Val Met His Glu Ala Leu His Asn His Tyr Thr

305 310 315 320

Gln Lys Ser Leu Ser Leu Ser Pro Gly Lys

325 330

<210> 24

<211> 330

<212> PRT

<213> Homo sapiens

<400> 24

Ala Ser Thr Lys Gly Pro Ser Val Phe Pro Leu Ala Pro Ser Ser Lys

1 5 10 15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45

Gly Val His Thr Phe Pro Ala Val Leu Gln Ser Ser Gly Leu Tyr Ser
            50                  55                  60

Leu Ser Ser Val Val Thr Val Pro Ser Ser Ser Leu Gly Thr Gln Thr
        65              70                  75                  80

Tyr Ile Cys Asn Val Asn His Lys Pro Ser Asn Thr Lys Val Asp Lys
                85                  90                  95

*1293

Lys Val Glu Pro Lys Ser Cys Asp Lys Thr His Thr Cys Pro Pro Cys

100                105                110

Pro Ala Pro Glu Leu Leu Gly Gly Pro Ser Val Phe Leu Phe Pro Pro

115                120                125

Lys Pro Lys Asp Thr Leu Met Ile Ser Arg Thr Pro Glu Val Thr Cys

130                135                140

Val Val Val Asp Val Ser His Glu Asp Pro Glu Val Lys Phe Asn Trp

145                150                155                160

Tyr Val Asp Gly Val Glu Val His Asn Ala Lys Thr Lys Pro Arg Glu

165 170 175

Glu Gln Tyr Asn Ser Thr Tyr Arg Val Val Ser Val Leu Thr Val Leu

180 185 190

His Gln Asp Trp Leu Asn Gly Lys Glu Tyr Lys Cys Lys Val Ser Asn

195 200 205

Lys Ala Leu Pro Ala Pro Ile Glu Lys Thr Ile Ser Lys Ala Lys Gly

210 215 220

Gln Pro Arg Glu Pro Gln Val Tyr Thr Leu Pro Pro Ser Arg Glu Glu

225 230 235 240

Met Thr Lys Asn Gln Val Ser Leu Thr Cys Leu Val Lys Gly Phe Tyr

245                          250                          255

Pro Ser Asp Ile Ala Val Glu Trp Glu Ser Asn Gly Gln Pro Glu Asn

260                          265                          270

Asn Tyr Lys Thr Thr Pro Pro Val Leu Asp Ser Asp Gly Ser Phe Phe

275                          280                          285

Leu Tyr Ser Lys Leu Thr Val Asp Lys Ser Arg Trp Gln Gln Gly Asn

290                          295                          300

Val Phe Ser Cys Ser Val Met His Glu Ala Leu His Asn His Tyr Thr

305                          310                          315                          320

Gln Lys Ser Leu Ser Leu Ser Pro Gly Lys

325                                    330

<210>      25

<211>      107

<212>      PRT

<213>      Homo sapiens

<400>      25

Arg Thr Val Ala Ala Pro Ser Val Phe Ile Phe Pro Pro Ser Asp Glu

1                    5                    10                    15

Gln Leu Lys Ser Gly Thr Ala Ser Val Val Cys Leu Leu Asn Asn Phe

20                    25                    30

Tyr Pro Arg Glu Ala Lys Val Gln Trp Lys Val Asp Asn Ala Leu Gln

35             40            45

Ser Gly Asn Ser Gln Glu Ser Val Thr Glu Gln Asp Ser Lys Asp Ser

50             55            60

Thr Tyr Ser Leu Ser Ser Thr Leu Thr Leu Ser Lys Ala Asp Tyr Glu

65             70            75            80

Lys His Lys Val Tyr Ala Cys Glu Val Thr His Gln Gly Leu Ser Ser

85             90            95

Pro Val Thr Lys Ser Phe Asn Arg Gly Glu Cys

100             105

<210>    26

<211>    107

<212>    PRT

<213>    Homo sapiens

<400>    26

Arg Ser Val Ala Ala Pro Ser Val Phe Ile Phe Pro Pro Ser Asp Glu

1                 5                    10                   15

Gln Leu Lys Ser Gly Thr Ala Ser Val Val Cys Leu Leu Asn Asn Phe

            20                   25                   30

Tyr Pro Arg Glu Ala Lys Val Gln Trp Lys Val Asp Asn Ala Leu Gln

            35                   40                   45

Ser Gly Asn Ser Gln Glu Ser Val Thr Glu Gln Asp Ser Lys Asp Ser

50 55 60

Thr Tyr Ser Leu Ser Ser Thr Leu Thr Leu Ser Lys Ala Asp Tyr Glu

65 70 75 80

Lys His Lys Val Tyr Ala Cys Glu Val Thr His Gln Gly Leu Ser Ser

85 90 95

*1391

Pro Val Thr Lys Ser Phe Asn Arg Gly Glu Cys

100 105

<210>    27

<211>    326

<212>    PRT

<213>    Homo sapiens

<400>    27

Ala Ser Thr Lys Gly Pro Ser Val Phe Pro Leu Ala Pro Cys Ser Arg

1                       5                       10                      15

Ser Thr Ser Glu Ser Thr Ala Ala Leu Gly Cys Leu Val Lys Asp Tyr

                20                      25                      30

Phe Pro Glu Pro Val Thr Val Ser Trp Asn Ser Gly Ala Leu Thr Ser

                35                      40                      45

Gly Val His Thr Phe Pro Ala Val Leu Gln Ser Ser Gly Leu Tyr Ser

50                         55                         60

Leu Ser Ser Val Val Thr Val Pro Ser Ser Asn Phe Gly Thr Gln Thr

65                    70                    75                    80

Tyr Thr Cys Asn Val Asp His Lys Pro Ser Asn Thr Lys Val Asp Lys

85                         90                         95

Thr Val Glu Arg Lys Cys Cys Val Glu Cys Pro Pro Cys Pro Ala Pro

100                       105                       110

Pro Val Ala Gly Pro Ser Val Phe Leu Phe Pro Pro Lys Pro Lys Asp

115                       120                       125

Thr Leu Met Ile Ser Arg Thr Pro Glu Val Thr Cys Val Val Val Asp

130                     135                     140

Val Ser His Glu Asp Pro Glu Val Gln Phe Asn Trp Tyr Val Asp Gly

145                     150                     155                     160

Val Glu Val His Asn Ala Lys Thr Lys Pro Arg Glu Glu Gln Phe Asn

165                     170                     175

Ser Thr Phe Arg Val Val Ser Val Leu Thr Val Val His Gln Asp Trp

180                     185                     190

Leu Asn Gly Lys Glu Tyr Lys Cys Lys Val Ser Asn Lys Gly Leu Pro

195                     200                     205

Ala Pro Ile Glu Lys Thr Ile Ser Lys Thr Lys Gly Gln Pro Arg Glu

210 215 220

Pro Gln Val Tyr Thr Leu Pro Pro Ser Arg Glu Glu Met Thr Lys Asn

225 230 235 240

Gln Val Ser Leu Thr Cys Leu Val Lys Gly Phe Tyr Pro Ser Asp Ile

245 250 255

Ser Val Glu Trp Glu Ser Asn Gly Gln Pro Glu Asn Asn Tyr Lys Thr

260 265 270

Thr Pro Pro Met Leu Asp Ser Asp Gly Ser Phe Phe Leu Tyr Ser Lys

275 280 285

Leu Thr Val Asp Lys Ser Arg Trp Gln Gln Gly Asn Val Phe Ser Cys

290                    295                    300

Ser Val Met His Glu Ala Leu His Asn His Tyr Thr Gln Lys Ser Leu

305                    310                    315                    320

Ser Leu Ser Pro Gly Lys

325

<210>     28

<211>     377

<212>     PRT

<213>     Homo sapiens

<400> 28

Ala Ser Thr Lys Gly Pro Ser Val Phe Pro Leu Ala Pro Cys Ser Arg

1               5                   10                  15

Ser Thr Ser Gly Gly Thr Ala Ala Leu Gly Cys Leu Val Lys Asp Tyr

            20                  25                  30

Phe Pro Glu Pro Val Thr Val Ser Trp Asn Ser Gly Ala Leu Thr Ser

            35                  40                  45

Gly Val His Thr Phe Pro Ala Val Leu Gln Ser Ser Gly Leu Tyr Ser

            50                  55                  60

Leu Ser Ser Val Val Thr Val Pro Ser Ser Ser Leu Gly Thr Gln Thr

            65                  70                  75                  80

Tyr Thr Cys Asn Val Asn His Lys Pro Ser Asn Thr Lys Val Asp Lys

85                    90                    95

Arg Val Glu Leu Lys Thr Pro Leu Gly Asp Thr Thr His Thr Cys Pro

100                   105                   110

Arg Cys Pro Glu Pro Lys Ser Cys Asp Thr Pro Pro Pro Cys Pro Arg

115                   120                   125

Cys Pro Glu Pro Lys Ser Cys Asp Thr Pro Pro Pro Cys Pro Arg Cys

130                   135                   140

Pro Glu Pro Lys Ser Cys Asp Thr Pro Pro Pro Cys Pro Arg Cys Pro

145                   150                   155                   160

Ala Pro Glu Leu Leu Gly Gly Pro Ser Val Phe Leu Phe Pro Pro Lys

165 170 175

Pro Lys Asp Thr Leu Met Ile Ser Arg Thr Pro Glu Val Thr Cys Val

180 185 190

Val Val Asp Val Ser His Glu Asp Pro Glu Val Gln Phe Lys Trp Tyr

195 200 205

Val Asp Gly Val Glu Val His Asn Ala Lys Thr Lys Pro Arg Glu Glu

210 215 220

Gln Tyr Asn Ser Thr Phe Arg Val Val Ser Val Leu Thr Val Leu His

225 230 235 240

Gln Asp Trp Leu Asn Gly Lys Glu Tyr Lys Cys Lys Val Ser Asn Lys

245 250 255

Ala Leu Pro Ala Pro Ile Glu Lys Thr Ile Ser Lys Thr Lys Gly Gln

260 265 270

Pro Arg Glu Pro Gln Val Tyr Thr Leu Pro Pro Ser Arg Glu Glu Met

275 280 285

Thr Lys Asn Gln Val Ser Leu Thr Cys Leu Val Lys Gly Phe Tyr Pro

290 295 300

Ser Asp Ile Ala Val Glu Trp Glu Ser Ser Gly Gln Pro Glu Asn Asn

305 310 315 320

Tyr Asn Thr Thr Pro Pro Met Leu Asp Ser Asp Gly Ser Phe Phe Leu

325                     330                     335

Tyr Ser Lys Leu Thr Val Asp Lys Ser Arg Trp Gln Gln Gly Asn Ile

340                     345                     350

Phe Ser Cys Ser Val Met His Glu Ala Leu His Asn Arg Phe Thr Gln

355                     360                     365

Lys Ser Leu Ser Leu Ser Pro Gly Lys

370                     375

<210>     29

<211>    327

<212>    PRT

<213>    Homo sapiens

<400>    29

Ala Ser Thr Lys Gly Pro Ser Val Phe Pro Leu Ala Pro Cys Ser Arg

1                5                10                15

Ser Thr Ser Glu Ser Thr Ala Ala Leu Gly Cys Leu Val Lys Asp Tyr

20                25                30

Phe Pro Glu Pro Val Thr Val Ser Trp Asn Ser Gly Ala Leu Thr Ser

35                40                45

Gly Val His Thr Phe Pro Ala Val Leu Gln Ser Ser Gly Leu Tyr Ser

50                55                60

Leu Ser Ser Val Val Thr Val Pro Ser Ser Ser Leu Gly Thr Lys Thr

65                70                75                80

Tyr Thr Cys Asn Val Asp His Lys Pro Ser Asn Thr Lys Val Asp Lys

85                90                95

Arg Val Glu Ser Lys Tyr Gly Pro Pro Cys Pro Ser Cys Pro Ala Pro

100                105                110

Glu Phe Leu Gly Gly Pro Ser Val Phe Leu Phe Pro Pro Lys Pro Lys

115                120                125

Asp Thr Leu Met Ile Ser Arg Thr Pro Glu Val Thr Cys Val Val Val

```
            130                    135                       140

Asp Val Ser Gln Glu Asp Pro Glu Val Gln Phe Asn Trp Tyr Val Asp

    145                150                   155                   160

Gly Val Glu Val His Asn Ala Lys Thr Lys Pro Arg Glu Glu Gln Phe

                   165                170                   175

Asn Ser Thr Tyr Arg Val Val Ser Val Leu Thr Val Leu His Gln Asp

                   180                185                   190

Trp Leu Asn Gly Lys Glu Tyr Lys Cys Lys Val Ser Asn Lys Gly Leu

                   195                200                   205

Pro Ser Ser Ile Glu Lys Thr Ile Ser Lys Ala Lys Gly Gln Pro Arg
```

210                    215                    220

Glu Pro Gln Val Tyr Thr Leu Pro Pro Ser Gln Glu Glu Met Thr Lys

225                    230                    235                    240

Asn Gln Val Ser Leu Thr Cys Leu Val Lys Gly Phe Tyr Pro Ser Asp

245                    250                    255

Ile Ala Val Glu Trp Glu Ser Asn Gly Gln Pro Glu Asn Asn Tyr Lys

260                    265                    270

Thr Thr Pro Pro Val Leu Asp Ser Asp Gly Ser Phe Phe Leu Tyr Ser

275                    280                    285

Arg Leu Thr Val Asp Lys Ser Arg Trp Gln Glu Gly Asn Val Phe Ser

290                    295                    300

Cys Ser Val Met His Glu Ala Leu His Asn His Tyr Thr Gln Lys Ser

305                    310                    315                    320

Leu Ser Leu Ser Leu Gly Lys

325

<210>    30

<211>    332

<212>    PRT

<213>    Artificial Sequence

<220>

<223>    Heavy chain constant region

<400>    30

Ala Ser Ala Lys Thr Thr Ala Pro Ser Val Tyr Pro Leu Ala Pro Val

1                   5                   10                  15

Cys Gly Asp Thr Thr Gly Ser Ser Val Thr Leu Gly Cys Leu Val Lys

                20                  25                  30

Gly Tyr Phe Pro Glu Pro Val Thr Leu Thr Trp Asn Ser Gly Ser Leu

                35                  40                  45

Ser Ser Gly Val His Thr Phe Pro Ala Val Leu Gln Ser Asp Leu Tyr

                50                  55                  60

Thr Leu Ser Ser Ser Val Thr Val Thr Ser Ser Thr Trp Pro Ser Gln

65     70     75     80

Ser Ile Thr Cys Asn Val Ala His Pro Ala Ser Ser Thr Lys Val Asp

85     90     95

Lys Lys Ile Glu Pro Arg Gly Pro Thr Ile Lys Pro Cys Pro Pro Cys

100     105     110

Lys Cys Pro Ala Pro Asn Leu Leu Gly Gly Pro Ser Val Phe Ile Phe

115     120     125

Pro Pro Lys Ile Lys Asp Val Leu Met Ile Ser Leu Ser Pro Ile Val

130     135     140

Thr Cys Val Val Val Asp Val Ser Glu Asp Asp Pro Asp Val Gln Ile

145                 150                 155                 160

Ser Trp Phe Val Asn Asn Val Glu Val His Thr Ala Gln Thr Gln Thr

                165                 170                 175

His Arg Glu Asp Tyr Asn Ser Thr Leu Arg Val Val Ser Ala Leu Pro

                180                 185                 190

Ile Gln His Gln Asp Trp Met Ser Gly Lys Glu Phe Lys Cys Lys Val

                195                 200                 205

Asn Asn Lys Asp Leu Pro Ala Pro Ile Glu Arg Thr Ile Ser Lys Pro

                210                 215                 220

Lys Gly Ser Val Arg Ala Pro Gln Val Tyr Val Leu Pro Pro Pro Glu

225                 230                 235                 240

Glu Glu Met Thr Lys Lys Gln Val Thr Leu Thr Cys Met Val Thr Asp

                245                 250                 255

Phe Met Pro Glu Asp Ile Tyr Val Glu Trp Thr Asn Asn Gly Lys Thr

                260                 265                 270

Glu Leu Asn Tyr Lys Asn Thr Glu Pro Val Leu Asp Ser Asp Gly Ser

                275                 280                 285

Tyr Phe Met Tyr Ser Lys Leu Arg Val Glu Lys Lys Asn Trp Val Glu

                290                 295                 300

Arg Asn Ser Tyr Ser Cys Ser Val Val His Glu Gly Leu His Asn His

305                      310                      315                      320

His Thr Thr Lys Ser Phe Ser Arg Thr Pro Gly Lys

                         325                      330

<210>    31

<211>    107

<212>    PRT

<213>    Artificial Sequence

<220>

<223>    Light chain constant region

<400>    31

Arg Thr Asp Ala Ala Pro Thr Val Ser Ile Phe Pro Pro Ser Ser Glu

1               5               10              15

Gln Leu Thr Ser Gly Gly Ala Ser Val Val Cys Phe Leu Asn Asn Phe

20              25              30

Tyr Pro Lys Asp Ile Asn Val Lys Trp Lys Ile Asp Gly Ser Glu Arg

35              40              45

Gln Asn Gly Val Leu Asn Ser Trp Thr Asp Gln Asp Ser Lys Asp Ser

50              55              60

Thr Tyr Ser Met Ser Ser Thr Leu Thr Leu Thr Lys Asp Glu Tyr Glu

65    70    75    80

Arg His Asn Ser Tyr Thr Cys Glu Ala Thr His Lys Thr Ser Thr Ser

85    90    95

Pro Ile Val Lys Ser Phe Asn Arg Asn Glu Cys

100    105

<210>  32

<211>  245

<212>  PRT

<213>  Artificial Sequence

<220>

<223>    Hybrid Fc Heavy region

<400>    32

Arg Asn Thr Gly Arg Gly Gly Glu Glu Lys Lys Lys Glu Lys Glu Lys

1                5                     10                    15

Glu Glu Gln Glu Glu Arg Glu Thr Lys Thr Pro Glu Cys Pro Ser His

                 20                    25                    30

Thr Gln Pro Leu Gly Val Phe Leu Phe Pro Pro Lys Pro Lys Asp Thr

                 35                    40                    45

Leu Met Ile Ser Arg Thr Pro Glu Val Thr Cys Val Val Val Asp Val

50          55          60

Ser Gln Glu Asp Pro Glu Val Gln Phe Asn Trp Tyr Val Asp Gly Val

65          70          75          80

Glu Val His Asn Ala Lys Thr Lys Pro Arg Glu Glu Gln Phe Asn Ser

85          90          95

*1748

Thr Tyr Arg Val Val Ser Val Leu Thr Val Leu His Gln Asp Trp Leu

100          105          110

Asn Gly Lys Glu Tyr Lys Cys Lys Val Ser Asn Lys Gly Leu Pro Ser

115          120          125

Ser Ile Glu Lys Thr Ile Ser Lys Ala Lys Gly Gln Pro Arg Glu Pro

130               135                    140

Gln Val Tyr Thr Leu Pro Pro Ser Gln Glu Glu Met Thr Lys Asn Gln

145               150                    155                    160

Val Ser Leu Thr Cys Leu Val Lys Gly Phe Tyr Pro Ser Asp Ile Ala

                 165                    170                    175

Val Glu Trp Glu Ser Asn Gly Gln Pro Glu Asn Asn Tyr Lys Thr Thr

                 180                    185                    190

Pro Pro Val Leu Asp Ser Asp Gly Ser Phe Phe Leu Tyr Ser Arg Leu

                 195                    200                    205

Thr Val Asp Lys Ser Arg Trp Gln Glu Gly Asn Val Phe Ser Cys Ser

210                215                220

Val Met His Glu Ala Leu His Asn His Tyr Thr Gln Lys Ser Leu Ser

225                230                235                240

Leu Ser Leu Gly Lys

245

<210>    33

<211>    990

<212>    DNA

<213>    Homo sapiens

<400>     33

gctagcacca agggcccatc ggtcttcccc ctggcgccct cctccaagag cacctctggg

60

ggcacagcgg ccctgggctg cctggtcaag gactacttcc ccgaaccggt gacggtgtcg

120

tggaactcag gggccctgac cagcggcgtg cacaccttcc cggctgtcct acagtcctca

180

ggactctact ccctcagcag cgtggtgacc gtgccctcca gcagcctggg cacccagacc

240

tacatctgca acgtgaatca caagcccagc aacaccaagg tggacaagaa agttgagccc

300

aaatcttgtg acaaaactca cacatgccca ccgtgcccag cacctgaact cctggggggga

360

ccgtcagtct tcctcttccc cccaaaaccc aaggacaccc tcatgatctc ccggacccct

420

gaggtcacat gcgtggtggt ggacgtgagc cacgaagacc ctgaggtcaa gttcaactgg

480

tacgtggacg gcgtggaggt gcataatgcc aagacaaagc cgcgggagga gcagtacaac

540

agcacgtacc gtgtggtcag cgtcctcacc gtcctgcacc aggactggct gaatggcaag

600

gagtacaagt gcaaggtctc caacaaagcc ctcccagccc ccatcgagaa aaccatctcc

660

aaagccaaag ggcagccccg agaaccacag gtgtacaccc tgcccccatc ccgggaggag

720

atgaccaaga accaggtcag cctgacctgc ctggtcaaag gcttctatcc cagcgacatc

780

gccgtggagt gggagagcaa tgggcagccg gagaacaact acaagaccac gcctcccgtg

840

ctggactccg acggctcctt cttcctctac agcaagctca ccgtggacaa gagcaggtgg

900

EP 4 286 412 A1

cagcagggga acgtcttctc atgctccgtg atgcatgagg ctctgcacaa ccactacacg

960

cagaagagcc tctccctgtc tccgggtaaa

990

<210>    34

<211>    321

<212>    DNA

<213>    Homo sapiens

<400>    34

agatctgtgg ctgcaccatc tgtcttcatc ttcccgccat ctgatgagca gttgaaatct

60

ggaactgcct ctgttgtgtg cctgctgaat aacttctatc ccagagaggc caaagtacag

120

tggaaggtgg ataacgccct ccaatcgggt aactcccagg agagtgtcac agagcaggac

180

agcaaggaca gcacctacag cctcagcagc accctgacgc tgagcaaagc agactacgag

240

aaacacaaag tctacgcctg cgaagtcacc catcagggcc tgagctcgcc cgtcacaaag

300

agcttcaaca ggggagagtg t

321

<210> 35

<211> 999

<212> DNA

<213> Artificial Sequence

<220>

<223> Heavy chain constant region

<400> 35

gctagcgcca aaacaacagc cccctccgta tacccccctag cccccgtatg cggcgacacc

60

accggctcct ccgtaaccct aggctgccta gtaaaaggct acttccccga acccgtaacc

120

ctaacctgga actccggctc cctatcctcc ggcgtacaca ccttccccgc cgtactacaa

180

tccgacctat acaccctatc ctcctccgta accgtaacct cctccacctg ccctcccaa

240

tccatcacct gcaacgtagc ccaccccgcc tcctccacca aagtagacaa aaaaatcgag

300

ccaaggggcc ccaccatcaa gccttgccct ccctgcaagt gccctgcccc caacctgctg

360

ggcggcccct ccgtgttcat cttccctcca aagatcaagg acgtgctgat gatctccctg

420

agccccatcg tgacctgcgt ggtggtggac gtgtccgagg acgaccctga cgtgcagatc

480

agctggttcg tgaacaacgt ggaggtgcac accgcccaga cccagaccca cagagaggac

540

tacaactcca ccctgagggt ggtgagcgcc ctgccaatcc agcaccagga ctggatgtcc

600

ggcaaggagt tcaagtgcaa ggtgaacaac aaggacctgc cagccccaat cgagaggacc

660

atcagcaagc ctaagggctc cgtgagggcc ccccaggtgt acgtgctgcc ccctcctgag

720

gaggagatga ccaagaagca ggtgaccctg acctgcatgg tgaccgactt catgcctgag

780

gacatctacg tggagtggac caacaacggc aagaccgagc tgaactacaa gaacaccgag

840

cctgtgctgg acagcgacgg cagctacttc atgtacagca agctgagggt ggagaagaag

900

aactgggtgg agagaaactc ctactcctgc tccgtggtgc acgagggcct gcacaaccac

960

cacaccacca agagcttctc cagaacccccc gggaagtga

999

<210>    36

<211>    321

<212>    DNA

<213>    Artificial Sequence

<220>

<223>    Light chain constant region

<400>    36

cgtacggatg ctgcaccaac tgtatccatc ttcccaccat ccagtgagca gttaacatct

60

ggaggtgcct cagtcgtgtg cttcttgaac aacttctacc ccaaagacat caatgtcaag

120

tggaagattg atggcagtga acgacaaaat ggcgtcctga acagttggac tgatcaggac

180

agcaaagaca gcacctacag catgagcagc accctcacgt tgaccaagga cgagtatgaa

240

cgacataaca gctatacctg tgaggccact cacaagacat caacttcacc cattgtcaag

300

agcttcaaca ggaatgagtg t

321

<210> 37

<211> 457

<212> PRT

<213> Artificial Sequence

<220>

<223> Heavy chain

<400> 37

Glu Val Gln Leu Leu Glu Ser Gly Gly Gly Leu Val Gln Pro Gly Gly

Ser Leu Arg Leu Ser Cys Ala Ala Ser Gly Phe Thr Phe Ser Asn Tyr

Ala Met Ser Trp Val Arg Gln Ala Pro Gly Lys Gly Leu Glu Trp Val

Ser Val Ile Ser His Gly Gly Gly Ser Thr Tyr Tyr Ala Asp Ser Val

Lys Gly Arg Phe Thr Ile Ser Arg Asp Asn Ser Lys Asn Thr Leu Tyr

Leu Gln Met Asn Ser Leu Arg Ala Glu Asp Thr Ala Val Tyr Tyr Cys

                        85                          90                          95

Ala Arg Val Ile Ser Asn Cys His Leu Gly Val Cys Tyr Tyr Ser Asn

                        100                         105                         110

Gly Met Asp Val Trp Gly Gln Gly Thr Leu Val Thr Val Ser Ser Ala

                        115                         120                         125

Lys Thr Thr Ala Pro Ser Val Tyr Pro Leu Ala Pro Val Cys Gly Asp

                        130                         135                         140

Thr Thr Gly Ser Ser Val Thr Leu Gly Cys Leu Val Lys Gly Tyr Phe

145                         150                         155                         160

Pro Glu Pro Val Thr Leu Thr Trp Asn Ser Gly Ser Leu Ser Ser Gly

165                    170                    175

Val His Thr Phe Pro Ala Val Leu Gln Ser Asp Leu Tyr Thr Leu Ser

180                    185                    190

Ser Ser Val Thr Val Thr Ser Ser Thr Trp Pro Ser Gln Ser Ile Thr

195                    200                    205

Cys Asn Val Ala His Pro Ala Ser Ser Thr Lys Val Asp Lys Lys Ile

210                    215                    220

Glu Pro Arg Gly Pro Thr Ile Lys Pro Cys Pro Pro Cys Lys Cys Pro

225                    230                    235                    240

Ala Pro Asn Leu Leu Gly Gly Pro Ser Val Phe Ile Phe Pro Pro Lys

245                     250                     255

Ile Lys Asp Val Leu Met Ile Ser Leu Ser Pro Ile Val Thr Cys Val

        260                     265                     270

Val Val Asp Val Ser Glu Asp Asp Pro Asp Val Gln Ile Ser Trp Phe

        275                     280                     285

Val Asn Asn Val Glu Val His Thr Ala Gln Thr Gln Thr His Arg Glu

        290                     295                     300

Asp Tyr Asn Ser Thr Leu Arg Val Val Ser Ala Leu Pro Ile Gln His

305                     310                     315                     320

Gln Asp Trp Met Ser Gly Lys Glu Phe Lys Cys Lys Val Asn Asn Lys

325                 330                 335

Asp Leu Pro Ala Pro Ile Glu Arg Thr Ile Ser Lys Pro Lys Gly Ser

340                 345                 350

Val Arg Ala Pro Gln Val Tyr Val Leu Pro Pro Pro Glu Glu Glu Met

355                 360                 365

Thr Lys Lys Gln Val Thr Leu Thr Cys Met Val Thr Asp Phe Met Pro

370                 375                 380

Glu Asp Ile Tyr Val Glu Trp Thr Asn Asn Gly Lys Thr Glu Leu Asn

385                 390                 395                 400

Tyr Lys Asn Thr Glu Pro Val Leu Asp Ser Asp Gly Ser Tyr Phe Met

405 410 415

Tyr Ser Lys Leu Arg Val Glu Lys Lys Asn Trp Val Glu Arg Asn Ser

420 425 430

Tyr Ser Cys Ser Val Val His Glu Gly Leu His Asn His His Thr Thr

435 440 445

Lys Ser Phe Ser Arg Thr Pro Gly Lys

450 455

<210> 38

<211> 457

<212> PRT

<213>    Artificial Sequence

<220>

<223>    Heavy chain

*2013

<400>    38

Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser Asn Tyr

            20                  25                  30

Ala Met Ser Trp Val Arg Gln Ala Pro Gly Lys Gly Leu Glu Trp Val

35           40          45

Ser Val Ile Ser His Gly Gly Gly Ser Thr Tyr Tyr Ala Asp Ser Val

50           55          60

Lys Gly Arg Phe Thr Ile Ser Arg Asp Asn Ser Lys Asn Thr Leu Tyr

65           70          75          80

Leu Gln Met Asn Ser Leu Arg Ala Glu Asp Thr Ala Val Tyr Tyr Cys

85           90          95

Ala Arg Val Ile Ser Asn Cys His Leu Gly Val Cys Tyr Tyr Ser Asn

100           105          110

Gly Met Asp Val Trp Gly Gln Gly Thr Leu Val Thr Val Ser Ser Ala

                    115                    120                    125

Ser Thr Lys Gly Pro Ser Val Phe Pro Leu Ala Pro Ser Ser Lys Ser

        130                    135                    140

Thr Ser Gly Gly Thr Ala Ala Leu Gly Cys Leu Val Lys Asp Tyr Phe

    145                150                155                160

Pro Glu Pro Val Thr Val Ser Trp Asn Ser Gly Ala Leu Thr Ser Gly

                165                    170                    175

Val His Thr Phe Pro Ala Val Leu Gln Ser Ser Gly Leu Tyr Ser Leu

                180                    185                    190

Ser Ser Val Val Thr Val Pro Ser Ser Ser Leu Gly Thr Gln Thr Tyr

195                     200                     205

Ile Cys Asn Val Asn His Lys Pro Ser Asn Thr Lys Val Asp Lys Lys

    210                     215                     220

Val Glu Pro Lys Ser Cys Asp Lys Thr His Thr Cys Pro Pro Cys Pro

225                     230                     235                     240

Ala Pro Glu Leu Leu Gly Gly Pro Ser Val Phe Leu Phe Pro Pro Lys

                    245                     250                     255

Pro Lys Asp Thr Leu Met Ile Ser Arg Thr Pro Glu Val Thr Cys Val

                    260                     265                     270

Val Val Asp Val Ser His Glu Asp Pro Glu Val Lys Phe Asn Trp Tyr

275　　　　　　　280　　　　　　　285

Val Asp Gly Val Glu Val His Asn Ala Lys Thr Lys Pro Arg Glu Glu

290　　　　　　　295　　　　　　　300

Gln Tyr Asn Ser Thr Tyr Arg Val Val Ser Val Leu Thr Val Leu His

305　　　　　　　310　　　　　　　315　　　　　　　320

Gln Asp Trp Leu Asn Gly Lys Glu Tyr Lys Cys Lys Val Ser Asn Lys

325　　　　　　　330　　　　　　　335

Ala Leu Pro Ala Pro Ile Glu Lys Thr Ile Ser Lys Ala Lys Gly Gln

340　　　　　　　345　　　　　　　350

Pro Arg Glu Pro Gln Val Tyr Thr Leu Pro Pro Ser Arg Glu Glu Met

355     360     365

Thr Lys Asn Gln Val Ser Leu Thr Cys Leu Val Lys Gly Phe Tyr Pro

370     375     380

Ser Asp Ile Ala Val Glu Trp Glu Ser Asn Gly Gln Pro Glu Asn Asn

385     390     395     400

Tyr Lys Thr Thr Pro Pro Val Leu Asp Ser Asp Gly Ser Phe Phe Leu

405     410     415

Tyr Ser Lys Leu Thr Val Asp Lys Ser Arg Trp Gln Gln Gly Asn Val

420     425     430

Phe Ser Cys Ser Val Met His Glu Ala Leu His Asn His Tyr Thr Gln

EP 4 286 412 A1

435                    440                    445

Lys Ser Leu Ser Leu Ser Pro Gly Lys

450                    455

<210>    39

<211>    218

<212>    PRT

<213>    Artificial Sequence

<220>

<223>    Light chain

*2111

<400>    39

Gln Leu Val Leu Thr Gln Pro Pro Ser Val Ser Ala Ala Pro Gly Gln

   1           5           10          15

Arg Val Ser Ile Ser Cys Ser Gly Ser Ser Ser Asn Ile Gly Asp Asn

        20          25          30

Tyr Val Ser Trp Tyr Gln Gln Val Pro Gly Ser Ala Pro Lys Leu Leu

        35          40          45

Ile Tyr Asp Asn Asn Lys Arg Pro Ser Gly Ile Pro Asp Arg Phe Ser

        50          55          60

Ala Ser Lys Ser Gly Thr Ser Ala Thr Leu Gly Ile Thr Gly Leu Gln

65                70                75                80

Thr Gly Asp Glu Ala Asp Tyr Tyr Cys Ala Thr Trp Asp Gly Ser Leu

                85                90                95

Ser Ala Gly Arg Val Phe Gly Gly Gly Thr Lys Leu Thr Val Leu Arg

           100            105           110

Ala Asp Ala Ala Pro Thr Val Ser Ile Phe Pro Pro Ser Ser Glu Gln

           115            120           125

Leu Thr Ser Gly Gly Ala Ser Val Val Cys Phe Leu Asn Asn Phe Tyr

           130            135           140

Pro Lys Asp Ile Asn Val Lys Trp Lys Ile Asp Gly Ser Glu Arg Gln

145                  150                  155                  160

Asn Gly Val Leu Asn Ser Trp Thr Asp Gln Asp Ser Lys Asp Ser Thr

                 165                  170                  175

Tyr Ser Met Ser Ser Thr Leu Thr Leu Thr Lys Asp Glu Tyr Glu Arg

                 180                  185                  190

His Asn Ser Tyr Thr Cys Glu Ala Thr His Lys Thr Ser Thr Ser Pro

                 195                  200                  205

Ile Val Lys Ser Phe Asn Arg Asn Glu Cys

             210                  215

<210>    40

*2158<211>    218

<212>    PRT

<213>    Artificial Sequence

<220>

<223>    Light chain

<400>    40

Gln Leu Val Leu Thr Gln Pro Pro Ser Val Ser Ala Ala Pro Gly Gln

Arg Val Ser Ile Ser Cys Ser Gly Ser Ser Ser Asn Ile Gly Asp Asn

20                          25                          30

Tyr Val Ser Trp Tyr Gln Gln Val Pro Gly Ser Ala Pro Lys Leu Leu

35                          40                          45

Ile Tyr Asp Asn Asn Lys Arg Pro Ser Gly Ile Pro Asp Arg Phe Ser

50                          55                          60

Ala Ser Lys Ser Gly Thr Ser Ala Thr Leu Gly Ile Thr Gly Leu Gln

65                          70                          75                          80

Thr Gly Asp Glu Ala Asp Tyr Tyr Cys Ala Thr Trp Asp Gly Ser Leu

85                          90                          95

Ser Ala Gly Arg Val Phe Gly Gly Gly Thr Lys Leu Thr Val Leu Arg

                    100                   105                   110

Thr Val Ala Ala Pro Ser Val Phe Ile Phe Pro Pro Ser Asp Glu Gln

                    115                   120                   125

Leu Lys Ser Gly Thr Ala Ser Val Val Cys Leu Leu Asn Asn Phe Tyr

                    130                   135                   140

Pro Arg Glu Ala Lys Val Gln Trp Lys Val Asp Asn Ala Leu Gln Ser

145                   150                   155                   160

Gly Asn Ser Gln Glu Ser Val Thr Glu Gln Asp Ser Lys Asp Ser Thr

                    165                   170                   175

Tyr Ser Leu Ser Ser Thr Leu Thr Leu Ser Lys Ala Asp Tyr Glu Lys

                    180                    185                    190

His Lys Val Tyr Ala Cys Glu Val Thr His Gln Gly Leu Ser Ser Pro

                    195                    200                    205

Val Thr Lys Ser Phe Asn Arg Gly Glu Cys

                    210                    215

<210>    41

<211>    1380

<212>    DNA

<213>    Artificial Sequence

<220>

<223>    Heavy chain

<400>    41

gaagttcagc tgcttgagtc tggcggagga ctggttcaac ctggcggaag cctgagactg 60

tcttgtgccg ccagcggctt caccttcagc aactacgcca tgagctgggt ccgacaggcc 120

cctggaaaag gccttgaatg ggtgtccgtg atctctcacg gcggaggcag cacctactac 180

gccgattctg tgaagggcag attcaccatc agccgggaca acagcaagaa caccctgtac 240

ctgcagatga acagcctgag agccgaggac accgccgtgt actattgcgc cagagtgatc

300

agcaactgcc acctgggcgt gtgctactac agcaacggca tggatgtgtg gggccagggc

360

acactggtta ccgttagttc tgctagcgcc aaaacaacag cccctccgt ataccccta

420

gcccccgtat gcggcgacac caccggctcc tccgtaaccc taggctgcct agtaaaaggc

480

tacttcccg aacccgtaac cctaacctgg aactccggct ccctatcctc cggcgtacac

540

accttccccg ccgtactaca atccgaccta tacaccctat cctcctccgt aaccgtaacc

600

tcctccacct ggccctccca atccatcacc tgcaacgtag cccaccccgc ctcctccacc

660

aaagtagaca aaaaaatcga gccaaggggc cccaccatca agccttgccc tccctgcaag

720

tgccctgccc ccaacctgct gggcggcccc tccgtgttca tcttccctcc aaagatcaag

780

gacgtgctga tgatctccct gagccccatc gtgacctgcg tggtggtgga cgtgtccgag

840

gacgaccctg acgtgcagat cagctggttc gtgaacaacg tggaggtgca caccgcccag

acccagaccc acagagagga ctacaactcc accctgaggg tggtgagcgc cctgccaatc

cagcaccagg actggatgtc cggcaaggag ttcaagtgca aggtgaacaa caaggacctg

ccagccccaa tcgagaggac catcagcaag cctaagggct ccgtgagggc cccccaggtg

tacgtgctgc cccctcctga ggaggagatg accaagaagc aggtgaccct gacctgcatg

gtgaccgact tcatgcctga ggacatctac gtggagtgga ccaacaacgg caagaccgag

1200

ctgaactaca agaacaccga gcctgtgctg gacagcgacg gcagctactt catgtacagc

1260

aagctgaggg tggagaagaa gaactgggtg gagagaaact cctactcctg ctccgtggtg

1320

cacgagggcc tgcacaacca ccacaccacc aagagcttct ccagaacccc cgggaagtga

1380

1380

<210>    42

<211>    1371

<212>    DNA

<213>    Artificial Sequence

<220>

<223>    Heavy chain

<400>    42

gaagttcagc tgcttgagtc tggcggagga ctggttcaac ctggcggaag cctgagactg

60

tcttgtgccg ccagcggctt caccttcagc aactacgcca tgagctgggt ccgacaggcc

120

cctggaaaag gccttgaatg ggtgtccgtg atctctacg gcggaggcag cacctactac

180

gccgattctg tgaagggcag attcaccatc agccgggaca acagcaagaa caccctgtac

240

ctgcagatga acagcctgag agccgaggac accgccgtgt actattgcgc cagagtgatc

300

agcaactgcc acctgggcgt gtgctactac agcaacggca tggatgtgtg gggccagggc

360

acactggtta ccgttagttc tgctagcacc aagggcccat cggtcttccc cctggcgccc

420

tcctccaaga gcacctctgg gggcacagcg gccctgggct gcctggtcaa ggactacttc

480

cccgaaccgg tgacggtgtc gtggaactca ggggccctga ccagcggcgt gcacaccttc

540

ccggctgtcc tacagtcctc aggactctac tccctcagca gcgtggtgac cgtgccctcc

600

agcagcctgg gcacccagac ctacatctgc aacgtgaatc acaagcccag caacaccaag

660

gtggacaaga aagttgagcc caaatcttgt gacaaaactc acacatgccc accgtgccca

720

gcacctgaac tcctgggggg accgtcagtc ttcctcttcc ccccaaaacc caaggacacc

780

ctcatgatct cccggacccc tgaggtcaca tgcgtggtgg tggacgtgag ccacgaagac

840

cctgaggtca agttcaactg gtacgtggac ggcgtggagg tgcataatgc caagacaaag

900

ccgcgggagg agcagtacaa cagcacgtac cgtgtggtca gcgtcctcac cgtcctgcac

960

caggactggc tgaatggcaa ggagtacaag tgcaaggtct ccaacaaagc cctcccagcc

1020

cccatcgaga aaaccatctc caaagccaaa gggcagcccc gagaaccaca ggtgtacacc

1080

ctgcccccat cccgggagga gatgaccaag aaccaggtca gcctgacctg cctggtcaaa

1140

ggcttctatc ccagcgacat cgccgtggag tgggagagca atgggcagcc ggagaacaac

1200

tacaagacca cgcctcccgt gctggactcc gacggctcct tcttcctcta cagcaagctc

1260

accgtggaca agagcaggtg gcagcagggg aacgtcttct catgctccgt gatgcatgag

1320

gctctgcaca accactacac gcagaagagc ctctccctgt ctccgggtaa a

1371

<210> 43

<211> 654

<212> DNA

<213> Artificial Sequence

<220>

<223> Light chain

<400> 43

caactggttc tgacacagcc tccaagcgtg tcagctgccc ctggacagag agtgtccatc

60

agctgtagcg gcagcagcag caacatcggc gacaactacg tgtcctggta tcagcaggtc

120

ccaggctctg cccctaagct gctgatctac gacaacaaca agcggcccag cggcatcccc

180

gatagatttt ctgccagcaa gagcggcacc agcgccacac tgggaattac aggactgcag

240

acaggcgacg aggccgacta ctattgtgcc acatgggatg gctccctgag cgccggaaga

300

gtttttggcg gaggcaccaa gctgaccgtg cttcgtacgg atgctgcacc aactgtatcc

360

atcttcccac catccagtga gcagttaaca tctggaggtg cctcagtcgt gtgcttcttg

420

aacaacttct accccaaaga catcaatgtc aagtggaaga ttgatggcag tgaacgacaa

480

aatggcgtcc tgaacagttg gactgatcag gacagcaaag acagcaccta cagcatgagc

540

agcaccctca cgttgaccaa ggacgagtat gaacgacata acagctatac ctgtgaggcc

600

actcacaaga catcaacttc acccattgtc aagagcttca acaggaatga gtgt

654

<210>      44

<211>      654

<212>      DNA

<213>      Artificial Sequence

<220>

<223>      Light chain

<400> 44

caactggttc tgacacagcc tccaagcgtg tcagctgccc ctggacagag agtgtccatc

60

agctgtagcg gcagcagcag caacatcggc gacaactacg tgtcctggta tcagcaggtc

120

ccaggctctg cccctaagct gctgatctac gacaacaaca agcggcccag cggcatcccc

180

gatagatttt ctgccagcaa gagcggcacc agcgccacac tgggaattac aggactgcag

240

acaggcgacg aggccgacta ctattgtgcc acatgggatg gctccctgag cgccggaaga

300

gtttttggcg gaggcaccaa gctgaccgtg cttagatctg tggctgcacc atctgtcttc

360

atcttcccgc catctgatga gcagttgaaa tctggaactg cctctgttgt gtgcctgctg

420

aataacttct atcccagaga ggccaaagta cagtggaagg tggataacgc cctccaatcg

480

ggtaactccc aggagagtgt cacagagcag gacagcaagg acagcaccta cagcctcagc

540

agcaccctga cgctgagcaa agcagactac gagaaacaca agtctacgc ctgcgaagtc

600

acccatcagg gcctgagctc gcccgtcaca aagagcttca acaggggaga gtgt

654

<210> 45

<211> 24

<212> DNA

<213> Artificial Sequence

<220>

<223> mouse Lrig-1 forward primer

<400> 45

gacggaattc agtgaggaga acct

24

<210> 46

<211> 24

<212> DNA

<213> Artificial Sequence

<220>

<223> mouse Lrig-1 reverse primer

<400> 46

caactggtag tggcagcttg tagg

24

<210> 47

<211> 24

<212> DNA

<213> Artificial Sequence

<220>

<223> mouse Lrig-2 forward primer

<400> 47

tcacaaggaa cattgtctga acca

24

<210>    48

<211>    24

<212>    DNA

<213>    Artificial Sequence

<220>

<223>    mouse Lrig-2 reverse primer

<400>    48

gcctgatcta acacatcctc ctca

24

<210>    49

<211>    24

<212>    DNA

<213>    Artificial Sequence

<220>

<223>    mouse Lrig-3 forward primer

<400>    49

cagcaccttg agctgaacag aaac

24

<210>    50

<211>    24

<212>    DNA

<213>    Artificial Sequence

<220>

<223>    mouse Lrig-3 reverse primer

<400>    50

ccagcctttg gtaatctcgg ttag

24

<210>    51

<211>    24

<212>    DNA

<213>    Artificial Sequence

<220>

<223>    mouse FOXP3 forward primer

<400>    51

ctttcaccta tcccaccctt atcc

24

<210>    52

<211>    24

<212>    DNA

<213>    Artificial Sequence

<220>

<223>    mouse FOXP3 reverse primer

<400>    52

attcatctac ggtccacact gctc

24

<210>    53

<211>    20

<212>    DNA

<213>    Artificial Sequence

<220>

<223>    ACTG1 forward primer

<400>    53

ggcgtcatgg tgggcatggg

20

<210>    54

<211>    20

<212>    DNA

<213>    Artificial Sequence

<220>

<223> ACTG1 reverse primer

<400> 54

atggcgtggg gaagggcgta

20

**Description**

Technical Field

The present invention relates to a novel binding molecule with excellent preventive, ameliorative or therapeutic effects on a brain and nervous system disease and uses thereof.

Background Art

In degenerative brain and nervous system diseases such as stroke, dementia, and Alzheimer's disease, deterioration in memory, attention, cognitive ability, emotion regulation, and the like is observed, which results from death of neuronal cells and atrophy of nerve branches. Nerve branch elongation in neuronal cells leads to increased neuroplasticity, and thus plays an important role in memory and learning functions of neural circuitry. Therefore, it is predicted that active ingredients, which promote nerve branch elongation and nerve regeneration in neuronal cells, have the potential to be developed as new therapeutic agents for degenerative brain and nervous system diseases.

As the aging population rapidly increases, the incidence of degenerative brain and nervous system diseases is also on the rise. Despite innovative advances in medicine, prophylactic and therapeutic methods for degenerative brain and nervous system diseases are not yet clearly established, and no drugs have been found which have a decisive effect. Currently, therapeutic agents and treatment methods for degenerative brain and nervous system diseases are being developed; however, they often exhibit side effects and toxicity due to long-term use, and only have an effect of alleviating symptoms rather than treating the disease. Therefore, there is an urgent need to develop a material capable of achieving treatment while having decreased side effects and toxicity.

Technical Problem

An object of the present invention is to provide a binding molecule that has excellent binding ability to the Lrig-1 protein present on the surface of regulatory T cells (Treg) and is excellent in preventing, ameliorating, or treating a brain and nervous system disease.

Another object of the present invention is to provide a nucleic acid molecule encoding said binding molecule according to the present invention.

Another object of the present invention is to provide an expression vector into which the nucleic acid molecule according to the present invention is inserted.

Another object of the present invention is to provide a host cell line transfected with the expression vector according to the present invention.

Another object of the present invention is to provide an antibody-drug conjugate according to the present invention.

Another object of the present invention is to provide a composition for preventing, ameliorating or treating a brain and nervous system disease comprising the binding molecule according to the present invention.

Another object of the present invention is to provide a pharmaceutical composition for preventing or treating a brain and nervous system disease comprising the antibody-drug conjugate according to the present invention as an active ingredient.

Another object of the present invention is to provide a pharmaceutical composition for preventing or treating a brain and nervous system disease comprising the chimeric antigen receptor (CAR) according to the present invention as an active ingredient.

Another object of the present invention is to provide a diagnostic composition and kit for diagnosing a brain and nervous system disease comprising the binding molecule according to the present invention, and a method for providing information for diagnosing a brain and nervous system disease.

Another object of the present invention is to provide a method for preventing or treating a brain and nervous system disease characterized by the binding molecule, antibody or fragment thereof, antibody-drug conjugate (ADC), or chimeric antigen receptor (CAR) according to the present invention.

However, the technical problem to be achieved by the present invention is not limited to the above-mentioned problems, and other problems that are not mentioned will be clearly understood by a skilled person in the art from the following description.

Solution to Problem

According to an embodiment of the present invention, there is provided a binding molecule that specifically binds to leucine-rich and immunoglobulin-like domains 1 (Lrig-1) protein present on the surface of regulatory T cells (Treg cells).

As used herein, the term "binding molecule" refers to a variable domain comprising an intact immunoglobulin that includes a monoclonal antibody, such as a chimeric, humanized, or human monoclonal antibody, or an immunoglobulin that binds to an antigen, for example, an immunoglobulin fragment that competes with intact immunoglobulins for binding to monomeric HA or trimeric HA of influenza A virus. Regardless of the structure, an antigen-binding fragment binds to the same antigen recognized by intact immunoglobulins. The antigen-binding fragment may include a peptide or polypeptide which contains, out of the amino acid sequence of the binding molecule, an amino acid sequence of two or more contiguous residues, 20 or more contiguous amino acid residues, 25 or more contiguous amino acid residues, 30 or more contiguous amino acid residues, 35 or more contiguous amino acid residues, 40 or more contiguous amino acid residues, 50 or more contiguous amino acid residues, 60 or more contiguous amino acid residues, 70 or more contiguous amino acid residues, 80 or more contiguous amino acid residues, 90 or more contiguous amino acid residues, 100 or more contiguous amino acid residues, 125 or more contiguous amino acid residues, 150 or more contiguous amino acid residues, 175 or more contiguous amino acid residues, 200 or more contiguous amino acid residues, or 250 or more contiguous amino acid residues. The term "antigen-binding fragment", in particular, includes Fab, F(ab'), F(ab')2, Fv, dAb, Fd, complementarity determining region (CDR) fragments, single-chain antibodies (scFvs), bivalent single-chain antibodies, single-chain phage antibodies, unibodies, diabodies, triabodies, tetrabodies, polypeptides containing one or more fragments of immunoglobulin which is sufficient for a particular antigen to bind to the polypeptide, and the like. The fragment may be produced synthetically or by enzymatic or chemical digestion of a complete immunoglobulin, or may be produced by genetic engineering methods using recombinant DNA techniques. Production methods are well known in the art.

In the present invention, the "Lrig-1 protein" is a transmembrane protein consisting of 1091 amino acids present on the surface of regulatory T cells, and is composed of leucine-rich repeats (LRRs) and three immunoglobulin-like domains on the extracellular or lumen side, a cell transmembrane sequence, and a cytoplasmic tail portion. The LRIG gene family includes LRIG1, LRIG2, and LRIG3, and the amino acids therebetween are highly conserved. The LRIG1 gene is highly expressed in normal skin and can be expressed in basal and hair follicle cells to regulate proliferation of epithelial stem cells. Therefore, the LRIG1 gene plays an important role in maintaining homeostasis of the epidermis, and its absence may develop psoriasis or skin cancer. It has been reported that in a case where chromosome 3p14.3 portion in which LRIG1 is located is cut off, there is a possibility of developing into cancer cells. In fact, it was identified that expression of LRIG1 is greatly decreased in renal cell carcinoma and cutaneous squamous cell carcinoma. Recently, it has been also found that Lrig-1 is expressed in only about 20 to 30% of cancers. On the other hand, for the purpose of the present invention, the Lrig-1 protein may be, but is not limited to, a protein present in humans or mice.

In the present invention, the Lrig-1 protein may be, but is not limited to, a human-derived polypeptide represented by SEQ ID NO: 1 or a mouse-derived polypeptide represented by SEQ ID NO: 3.

In addition, in the present invention, the Lrig-1 protein represented by SEQ ID NO: 1 may be encoded by a polynucleotide represented by SEQ ID NO: 2, but is not limited thereto.

In addition, in the present invention, the Lrig-1 protein represented by SEQ ID NO: 3 may be encoded by a polynucleotide represented by SEQ ID NO: 4, but is not limited thereto.

In the present invention, the binding molecule may be a binding molecule, comprising:

a heavy chain variable region that contains a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and

a light chain variable region that contains a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

In the present invention, the binding molecule may be a binding molecule, comprising a heavy chain variable region consisting of the amino acid sequence represented by SEQ ID NO: 11; and
a light chain variable region consisting of the amino acid sequence represented by SEQ ID NO: 12.

In the present invention, the binding molecule may be a binding molecule, comprising:

a heavy chain variable region that contains a heavy chain CDR1 encoded by the nucleotide sequence represented by SEQ ID NO: 13, a heavy chain CDR2 encoded by the nucleotide sequence represented by SEQ ID NO: 14, and a heavy chain CDR3 encoded by the nucleotide sequence represented by SEQ ID NO: 15; and

a light chain variable region that contains a light chain CDR1 encoded by the nucleotide sequence represented by SEQ ID NO: 16, a light chain CDR2 encoded by the nucleotide sequence represented by SEQ ID NO: 17, and a light chain CDR3 encoded by the nucleotide sequence represented by SEQ ID NO: 18.

In the present invention, the binding molecule may be binding molecule, comprising:

a heavy chain variable region encoded by the nucleotide sequence represented by SEQ ID NO: 19; and

a light chain variable region encoded by the nucleotide sequence represented by SEQ ID NO: 20.

In the present invention, the binding molecule may further comprise a fragment crystallization (Fc) region or a constant region. Here, the Fc region may be an Fc region of an IgA, IgD, IgE, IgM, IgG1, IgG2, IgG3, or IgG4 antibody, or may be derived therefrom. Alternatively, the Fc region may be a hybrid Fc region.
In the present invention, the Fc region may be an Fc region of a mammalian-derived IgA, IgD, IgE, IgM, IgG1, IgG2, IgG3, or IgG4 antibody, and may preferably be an Fc region of a human-derived IgA, IgD, IgE, IgM, IgG1, IgG2, IgG3, or IgG4 antibody. However, the Fc region is not limited thereto.
As an example of the present invention, the constant region may be a mouse-derived IgG2a constant region represented by SEQ ID NO: 21, but is not limited thereto.
As an example of the present invention, the constant region may be a mouse-derived immunoglobulin kappa constant region represented by SEQ ID NO: 22, but is not limited thereto.
As an example of the present invention, the constant region may be a human-derived IgG1 constant region represented by SEQ ID NO: 23 or 24, but is not limited thereto.
As an example of the present invention, the constant region may be a human-derived immunoglobulin kappa constant region represented by SEQ ID NO: 25 or 26, but is not limited thereto.
As an example of the present invention, the constant region may be a human-derived IgG2 constant region represented by SEQ ID NO: 27, but is not limited thereto.
As an example of the present invention, the constant region may be a human-derived IgG3 constant region represented by SEQ ID NO: 28, but is not limited thereto.
As an example of the present invention, the constant region may be a human-derived IgG4 constant region represented by SEQ ID NO: 29, but is not limited thereto.
As an example of the present invention, the constant region may be a constant region represented by SEQ ID NO: 30, but is not limited thereto.
As an example of the present invention, the constant region may be a constant region represented by SEQ ID NO: 31, but is not limited thereto.
As an example of the present invention, the Fc region may be a human-derived immunoglobulin lambda constant region, but is not limited thereto.
In the present invention, the "hybrid Fc" may be derived from a combination of human IgG subclasses or a combination of human IgD and IgG. In a case where the hybrid Fc binds to a biologically active molecule, polypeptide, or the like, the hybrid Fc has effects of not only increasing a serum half-life of the biologically active molecule, but also increasing an expression level of the polypeptide when a nucleotide sequence encoding the Fc-polypeptide fusion protein is expressed.
As an example of the present invention, the hybrid Fc region may be hybrid Fc represented by SEQ ID NO: 32, but is not limited thereto.
In the binding molecule of the present invention, the Fc or constant region may be linked, via a linker, to the variable region. Here, the linker may be linked to the C-terminus of the Fc or constant region, and the N-terminus of the binding molecule of the present invention may be linked to the linker. However, the present invention is not limited thereto.
In the present invention, the "linker" may contain a sequence that can be cleaved by an enzyme that is overexpressed in a tissue or cell having a target disease. In a case where the linker may be cleaved by the overexpressed enzyme as described above, it is possible to effectively prevent activity of a polypeptide from decreasing due to the Fc or constant region. In the present invention, an example of the linker may be preferably a peptide linker consisting of 33 amino acids located in the 282nd to 314th portion of human albumin which is most abundantly present in the blood, and more preferably a peptide linker consisting of 13 amino acids located in the 292nd to 304th portion of the human albumin. Such portions are portions which are mostly exposed to the outside in three-dimensional structure, and thus have a minimum possibility of inducing an immune response in the body. However, the linker is not limited thereto.
The binding molecule of the present invention may further comprise a heavy chain constant region consisting of an amino acid sequence selected from the group consisting of SEQ ID NOs: 21, 23, 24, 27, 28, 29, 30, and 32.
The binding molecule of the present invention may further comprise a light chain constant region consisting of the amino acid sequence represented by SEQ ID NO: 22, 25, 26, and 31.
The binding molecule of the present invention may further comprise:

a heavy chain constant region consisting of the amino acid sequence represented by SEQ ID NO: 21; and

a light chain constant region consisting of the amino acid sequence represented by SEQ ID NO: 22.

The binding molecule of the present invention may further comprise:

a heavy chain constant region represented by an amino acid sequence represented by SEQ ID NO: 23, 24, 27, 28, 29, and 30; and

a light chain constant region represented by an amino acid sequence represented by SEQ ID NO: 25, 26, and 31.

The binding molecule of the present invention may further comprise:

a heavy chain constant region consisting of the amino acid sequence represented by SEQ ID NO: 24; and

a light chain constant region consisting of the amino acid sequence represented by SEQ ID NO: 26.

The binding molecule of the present invention may further comprise:

a heavy chain constant region consisting of the amino acid sequence represented by SEQ ID NO: 30; and

a light chain constant region consisting of the amino acid sequence represented by SEQ ID NO: 31.

The binding molecule of the present invention may further comprise:
a heavy chain constant region consisting of the amino acid sequence represented by SEQ ID NO: 32.
The binding molecule of the present invention may further comprise:

a heavy chain constant region encoded by the nucleotide sequence represented by SEQ ID NO: 33 or 35; and

a light chain constant region encoded by the nucleotide sequence represented by SEQ ID NO: 34 or 36.

The binding molecule of the present invention may be a binding molecule comprising a heavy chain consisting of the amino acid sequence represented by SEQ ID NO: 37 or 38 and a light chain consisting of the amino acid sequence represented by SEQ ID NO: 39 or 40.
The binding molecule of the present invention may be a binding molecule comprising a heavy chain consisting of the amino acid sequence represented by SEQ ID NO: 37 and a light chain consisting of the amino acid sequence represented by SEQ ID NO: 39.
The binding molecule of the present invention may be a binding molecule comprising a heavy chain consisting of the amino acid sequence represented by SEQ ID NO: 38 and a light chain consisting of the amino acid sequence represented by SEQ ID NO: 40.
The binding molecule of the present invention may be a binding molecule comprising a heavy chain encoded by the nucleotide sequence represented by SEQ ID NO: 41 or 43 and a light chain encoded by the nucleotide sequence represented by SEQ ID NO: 42 or 44.
The binding molecule of the present invention may be a binding molecule comprising a heavy chain encoded by the nucleotide sequence represented by SEQ ID NO: 41 and a light chain encoded by the nucleotide sequence represented by SEQ ID NO: 42.
The binding molecule of the present invention may be a binding molecule comprising a heavy chain encoded by the nucleotide sequence represented by SEQ ID NO: 43 and a light chain encoded by the nucleotide sequence represented by SEQ ID NO: 44.
The binding molecule of the present invention is characterized by being an antibody or a fragment thereof, but is not limited thereto. The antibody includes all of a monoclonal antibody, a full-length antibody, or an antibody fragment which is a portion of an antibody, has the ability to bind to Lrig-1 protein, and can compete with the binding molecule of the present invention in binding to an epitope on Lrig-1.
As used herein, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surface of regulatory T cells. Preferably, the antibody may specifically recognize the leucine-rich region or immunoglobulin-like domain of the Lrig-1 protein, but is not limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

In addition, as used herein, the term "monoclonal antibody" refers to an antibody molecule of a single molecular composition which is obtained from substantially the same antibody population, and exhibits single binding specificity and affinity for a particular epitope.

In the present invention, the "full-length antibody" has a structure with two full-length light chains and two full-length heavy chains in which each light chain is linked to a heavy chain by a disulfide bond, and includes IgA, IgD, IgE, IgM, and IgG. The IgG includes, as subtypes thereof, IgG1, IgG2, IgG3, and IgG4.

In addition, as used herein, the term "antigen fragment" refers to a fragment that retains an antigen-binding function, and includes Fab, Fab', $F(ab')_2$, Fv, and the like. The Fab has a structure with variable regions of light and heavy chains, a constant region of the light chain, and a first constant region (CH1 domain) of the heavy chain, and has one antigen-binding site. In addition, Fab' is different from Fab in that Fab' has a hinge region containing at least one cysteine residue at the C-terminus of the heavy chain CH1 domain. $F(ab')_2$ antibodies are produced with cysteine residues at the hinge region of Fab' forming a disulfide bond. Fv (variable fragment) refers to the smallest antibody fragment having only a heavy chain variable region and a light chain variable region. Double-chain Fv (dsFv) is configured to be such that a heavy chain variable region and a light chain variable region are linked to each other by a disulfide bond, and single-chain Fv (scFv) is configured to be such that a heavy chain variable region and a light chain variable region are covalently linked to each other, in general, via a peptide linker. The antibody fragment may be obtained as Fab or $F(ab')_2$ fragment in a case where a proteolytic enzyme, for example, papain or pepsin is used, and may be produced through a genetic recombinant technique.

In addition, in the present invention, the antibody may be, but is not limited to, a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof. In the present invention, the "chimeric antibody" is an antibody which is obtained by recombination of a variable region of a mouse antibody and a constant region of a human antibody, and has a greatly improved immune response as compared with the mouse antibody.

In addition, as used herein, the term "humanized antibody" refers to an antibody obtained by modifying a protein sequence of an antibody derived from a non-human species so that the protein sequence is similar to an antibody variant naturally produced in humans. For example, the humanized antibody may be prepared as follows. Mouse-derived CDRs may be recombined with a human antibody-derived FR to prepare a humanized variable region, and the humanized variable region may be recombined with a constant region of a preferred human antibody to prepare a humanized antibody.

In the present invention, the binding molecule may be provided as a bispecific antibody or a bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and the bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding another target protein may be an antigen-binding domain capable of binding to a protein other than Lrig-1 protein, for example, but not limited to, PD-1 or a cell surface receptor. However, the antigen-binding domain is not limited thereto.

The bispecific antibody and the bispecific antigen-binding fragment according to the present invention may be provided in any suitable format. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or F(ab')2-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). The bispecific antibody and the bispecific antigen-binding fragment according to the invention may be designed and prepared by those skilled in the art.

A method for producing the bispecific antibody in the present invention comprises forming a reducing disulfide or non-reducing thioether bond, and chemical crosslinking of an antibody or antibody fragment. For example, N-succinimidyl-3-(-2-pyridyldithio)-propionate (SPDP) may be used, for example, for chemically crosslinking a Fab fragment through an SH-group at the hinge region, to generate a disulfide-linked bispecific F(ab)2 heterodimer.

In addition, an alternative method for producing the bispecific antibody in the present invention comprises fusing an antibody-producing hybridoma with, for example, polyethylene glycol, to produce quadroma cells capable of secreting bispecific antibodies.

The bispecific antibody and the bispecific antigen-binding fragment according to the invention may also be, for example, recombinantly produced by expression from a nucleic acid construct that encodes a polypeptide for an antigen-binding molecule.

For example, a DNA construct that contains a sequence encoding light and heavy chain variable domains for two antigen-binding domains (that is, light and heavy chain variable domains for an antigen-binding domain capable of binding to PD-1, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, *in vitro*) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity matured antibody may be produced by a procedure known in the art.

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

In introducing variations, the hydropathic index of amino acids may be considered. Each amino acid has been assigned hydropathic index depending on its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). The hydropathic amino acid index is very important in conferring the interactive biological function on a protein. It is known that substitution with an amino acid having similar hydropathic index allows a protein to retain similar biological activity. In a case where variations are introduced with reference to the hydropathic index, substitutions are made between amino acids that exhibit a hydropathic index difference of preferably within $\pm$ 2, more preferably within $\pm$ 1, and even more preferably within $\pm$ 0.5.

Meanwhile, it is also well known that substitutions between amino acids having similar hydrophilicity values result in proteins with equivalent biological activity. As disclosed in US Pat. No. 4,554,101, respective amino acid residues have been assigned the following hydrophilicity values: arginine (+3.0); lysine (+3.0); aspartate (+3.0 $\pm$ 1); glutamate (+3.0 $\pm$ 1); serine (+0.3); asparagine (+0.2); glutamine (+0.2); glycine (0); threonine (-0.4); proline (-0.5 $\pm$ 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In a case where variations are introduced with reference to the hydrophilicity values, substitutions may be made between amino acids that exhibit a hydrophilicity value difference of preferably within $\pm$ 2, more preferably within $\pm$ 1, and even more preferably within $\pm$ 0.5.

Amino acid exchanges in proteins which do not entirely alter activity of a molecule are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Gln/Glu.

Given the above-described variations with biologically equivalent activity, it is interpreted that the binding molecule of the present invention also includes sequences that exhibit substantial identity with the sequences listed in the Sequence Listing.

As used herein, the term "substantial identity" refers to a sequence showing at least 61% homology, more preferably 70% homology, even more preferably 80% homology, and most preferably 90% homology when the sequence of the present invention is aligned with any other sequence so that they maximally correspond to each other, and the aligned sequence is analyzed by using an algorithm typically used in the art. Alignment methods for comparison of sequences are known in the art. NCBI Basic Local Alignment Search Tool (BLAST) is accessible from the National Center for Biological Information (NBCI), or the like, and may be used in conjunction with sequencing programs, such as blastp, blasm, blastx, tblastn, and tblastx, on the internet. BLAST is accessible at http://www.ncbi.nlm.nih.gov/BLASTI. Sequence homology comparison methods using this program can be identified online (http://www.ncbi.nlm.nih.gov/BLAST/blast_help.html).

In the present invention, the binding molecule, preferably the antibody, may be produced by a conventional method for producing an antibody, and may be produced by affinity maturation.

As used herein, the term "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process that occurs in nature.

The binding molecule, preferably the antibody, provided in the present invention can effectively prevent, ameliorate, or treat brain and nervous system diseases, in particular, neurodegenerative diseases or neuroinflammatory diseases.

According to another embodiment of the present invention, there is provided a nucleic acid molecule encoding the binding molecule provided in the present invention.

The nucleic acid molecule of the present invention includes all nucleic acid molecules obtained by translating the amino acid sequences of the binding molecules provided in the present invention to polynucleotide sequences, as known to those skilled in the art. Therefore, various polynucleotide sequences may be prepared by an open reading frame (ORF), and all of these polynucleotide sequences are also included in the nucleic acid molecule of the present invention.

The nucleic acid molecule of the present invention may comprise a heavy chain encoded by the nucleotide sequence represented by SEQ ID NO: 41 or 43 and a light chain encoded by the nucleotide sequence represented by SEQ ID NO: 42 or 44.

The nucleic acid molecule of the present invention may comprise a heavy chain encoded by the nucleotide sequence represented by SEQ ID NO: 41 and a light chain encoded by the nucleotide sequence represented by SEQ ID NO: 42.

The nucleic acid molecule of the present invention may comprise a heavy chain encoded by the nucleotide sequence represented by SEQ ID NO: 43 and a light chain encoded by the nucleotide sequence represented by SEQ ID NO: 44.

According to another embodiment of the present invention, there is provided an expression vector into which the isolated nucleic acid molecule provided in the present invention is inserted.

In the present invention, the "vector" is a nucleic acid molecule capable of transporting another nucleic acid linked thereto. One type of vector is a "plasmid," which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication and episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, $Q\mu\mu$, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In order to facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

According to another embodiment of the present invention, there is provided a host cell line transfected with the expression vector provided in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell line is available.

According to another embodiment of the present invention, there is provided an antibody-drug conjugate (ADC) comprising the antibody provided in the present invention and a drug.

As used herein, the term "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an $\alpha$-amine group at the N-terminus of the heavy and/or light chain of the antibody.

In the present invention, examples of the reactive group capable of reacting and crosslinking with the $\alpha$-amine group are not particularly limited in terms of type as long as the reactive group can react and crosslink with an $\alpha$-amine group at the N-terminus of a heavy or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

As used herein, the term "drug" may mean any substance having a certain biological activity for a cell, which is a concept including DNA, RNA, or a peptide. The drug may be in a form which contains a reactive group capable of reacting and crosslinking with an $\alpha$-amine group, and also includes a form which contains a reactive group capable of reacting and crosslinking with an $\alpha$-amine group and to which a linker is linked.

According to another embodiment of the present invention, there is provided a pharmaceutical composition for preventing or treating a brain and nervous system disease comprising, as an active ingredient, a chimeric antigen receptor (CAR) comprising an antigen-specific binding domain, a linking domain and a CD3 zeta ($\zeta$) signaling domain.

As used herein, the term "chimeric antigen receptor" or "CAR" refers to an engineered receptor comprising an extracellular antigen binding domain and an intracellular signaling domain. Although the most common type of CAR comprises a single chain variable fragment (scFv) derived from a monoclonal antibody fused to the transmembrane and intracellular domains of a T cell co-receptor, such as the CD3 zeta ($\zeta$) chain, the present invention is not limited to these domains. Rather, the "chimeric antigen receptor" or "CAR" as used herein refers to any receptor engineered to express any intracellular signaling molecule and any extracellular antigen binding domain fused or linked thereto. In the present invention, the binding domain may comprise a single chain variable fragment (scFv) capable of specifically recognizing the Lrig-1 protein. In the present invention, the term "single chain variable fragment" or "scFv" refers to a fusion protein of a variable heavy chain (VH) and a variable light chain (VL) of an antibody by a peptide linker between VL and VH.

In addition, in the present invention, the VH domain and the VL domain may be connected through a flexible linker. In the present invention, the flexible linker may be a glycine/serine linker of about 10 to 30 amino acids (e.g., 30, 25, 20, 19, 18, 17, 16, 15, 14, 13, 12, 11, 10, 9, 8, 7, 6, or 5 amino acids), preferably 15 amino acids in length. In the present invention, the linker length can act as an important determining site for chimeric antigen receptors, so a linker shorter than the above range can increase affinity, but can also cause intracellular multimer formation to impair CAR expression, while linkers longer than this range may reduce antigen affinity by moving the VL and VH CDRs farther apart in proximity.

The chimeric antigen receptor of the present invention may further comprise at least one of a hinge region (or spacer) and a signaling domain. In the present invention, the hinge region is a region connecting antigen-binding domain and the transmembrane domain, and is also called a "spacer," and has the purpose of extending the antigen-binding domain from the T cell membrane or the NK cell membrane. In the present invention, the hinge region can be obtained from any suitable sequence from any genus, including, for example, human or parts thereof, or may include CD8, CD28, 4-1BB, OX40, all or part of the CD3 zeta ($\zeta$) chain, T cell receptor $\alpha$ or $\beta$ chain, CD28, CD3$\epsilon$, CD45, CD4, CD5, CD8, CD9, CD16, CD22, CD33, CD37, CD64, CD80, CD86, CD134, CD137, ICOS, CD154, functional derivatives thereof, or combinations thereof commonly used in the art, but is not limited thereto. In addition, the hinge region may comprise one selected from immunoglobulins (e.g., IgG1, IgG2, IgG3, IgG4, and IgD) without being limited to immunoglobulins, but is not limited thereto.

In the present invention, the signaling domain refers to a part of a chimeric antigen receptor found or engineered to be found inside a T cell. The signaling domain may or may not comprise a transmembrane that serves to fix the chimeric antigen receptor in the plasma membrane of T cells. The transmembrane domain and the signaling domain may be derived from the same protein (e.g., CD3 zeta (ζ) molecule), or the transmembrane domain and the signaling domain may be derived from different proteins (e.g., transmembrane domain of CD28 and intracellular signaling domain of CD3 zeta (ζ) molecule, or vice versa).

In the present invention, the transmembrane domain comprises, for example, T cell receptor α or β chain, all or part of the CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, but is not limited thereto. The costimulatory domain may comprise 4-1BB (CD137), OX40, CD27, CD28, CD30, CD40, PD-1, CD2, CD7, CD258, natural killer group 2 member C (NKG2C), natural killer group 2 member (NKG2D), B7-H3, CD83, ICAM-1, ligands that bind to LFA-1 (CD11a/CD18) or ICOS, active fragments thereof, functional derivatives thereof, or a functional signaling domain derived from a polypeptide comprising a combination thereof, but is not limited thereto.

In the present invention, the signaling domain may comprise a functional signaling domain derived from polypeptides comprising all or part of CD3 zeta (ζ), common FcR gamma (FcER1G), FcgammaRIIIa, FcRbeta (Fc epsilon lip), CD3gamma, CD3delta, CD3 epsilon, CD79a, CD79b, DNAX-activating protein 10 (DAP10), DNAX-activating protein 12 (DAP12), an active fragment thereof, a functional derivative thereof, or a combination thereof, but is not limited thereto, and such signaling domains are known in the art.

According to one embodiment of the present invention, in the chimeric antigen receptor (CAR) comprising an antigen-specific binding domain, a linking domain and a CD3 zeta (ζ) signaling domain, there is provided a chimeric antigen receptor wherein the specific binding domain is a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

In the present invention, the drug may include any drug regardless of type as long as the drug can treat brain and nervous system diseases, in particular, neurodegenerative diseases or neuroinflammatory diseases.

According to another embodiment of the present invention, the present invention relates to a composition for preventing, ameliorating, or treating a brain and nervous system disease comprising the binding molecule, nucleic acid molecule, expression vector, host cell line, or antibody-drug conjugate (ADC) of the present invention as an active ingredient.

The brain and nervous system disease to be prevented, ameliorated, or treated by the composition provided in the present invention may be a neurodegenerative disease or neuroinflammatory disease.

In the present invention, the "neurodegenerative disease" may refer to a disease caused by decreased function or loss of neurons, and the "neuroinflammatory disease" may refer to a disease caused by excessive inflammatory responses in the nervous system. As a specific example, the neurodegenerative disease or neuroinflammatory disease in the present invention may be selected from, but is not limited to, the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

In addition, the composition provided in the present invention may be used as a pharmaceutical composition or a food composition. However, the present invention is not limited thereto.

In the present invention, the "prevention" may include, without limitation, any act of blocking symptoms caused by a neurodegenerative disease or neuroinflammatory disease, or suppressing or delaying the symptoms, using the composition of the present invention.

In the present invention, the "treatment" or "amelioration" may include, without limitation, any act capable of ameliorating or beneficially altering symptoms caused by a neurodegenerative disease or neuroinflammatory disease, using the composition of the present invention.

In the present invention, the pharmaceutical composition may be characterized by being in the form of capsules, tablets, granules, injections, ointments, powders, or beverages, and the pharmaceutical composition may be characterized by being targeted to humans.

The pharmaceutical composition of the present invention may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, frequency of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

The food composition that includes the composition of the present invention may be prepared in the form of various foods, for example, beverages, gums, tea, vitamin complexes, powders, granules, tablets, capsules, confections, rice cakes, bread, and the like. The food composition of the present invention is composed of a plant extract having little toxicity and side effects, and thus can be used without worries in a case of being ingested for a long time for preventive purposes.

When the composition of the present invention is included in the food composition, it may be added in an amount corresponding to a rate of 0.1% to 50% of the total weight.

Here, in a case where the food composition is prepared in the form of beverages, there is no particular limitation except that the beverage contains the food composition at an indicated proportion, and the beverage may contain various flavoring agents, natural carbohydrates, or the like as additional ingredients, similarly to conventional beverages. That is, examples of the natural carbohydrates may include monosaccharides such as glucose, disaccharides such as fructose, polysaccharides such as sucrose, conventional sugars such as dextrin and cyclodextrin, and sugar alcohol such as xylitol, sorbitol, and erythritol. Examples of the flavoring agents may include natural flavoring agents (thaumatin, stevia extracts (such as rebaudioside A), glycyrrhizin, and the like) and synthetic flavoring agents (saccharin, aspartame, and the like).

In addition, the food composition of the present invention may contain various nutrients, vitamins, minerals (electrolytes), flavorings such as synthetic flavorings and natural flavorings, colorants, pectic acid and salts thereof, alginic acid and salts thereof, organic acids, protective colloidal thickeners, pH adjusting agents, stabilizers, preservatives, glycerin, alcohol, carbonizing agents used in carbonated beverages, and the like.

These ingredients may be used individually or in combination. A proportion of such additives is not so important, and is generally selected from the range of 0.1 to about 50 parts by weight per 100 parts by weight of the composition of the present invention.

According to still yet another embodiment of the present invention, there is provided a method for preventing, ameliorating, or treating a brain and nervous system disease, comprising a step of administering, to an individual in need thereof, the binding molecule provided in the present invention; a nucleic acid molecule encoding the binding molecule; an expression vector into which the nucleic acid molecule is inserted; a host cell line transfected with the expression vector; or the antibody-drug conjugate (ADC) provided in the present invention.

In the present invention, the "individual" is an individual suspected of developing a brain and nervous system disease, and the individual suspected of developing a brain and nervous system disease means a mammal, such as mice and domestic animals, including humans, who has developed or is likely to develop the disease in question. However, any individual, who is treatable with the active substance provided in the present invention, is included therein without limitation.

The brain and nervous system disease treated by the method of the present invention may be a neurodegenerative disease or neuroinflammatory disease. As a specific example, the neurodegenerative disease or neuroinflammatory disease in the present invention may be selected from, but is not limited to, the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

The method of the present invention may comprise a step of administering, in a pharmaceutically effective amount, the binding molecule provided in the present invention; a nucleic acid molecule encoding the binding molecule; an expression vector into which the nucleic acid molecule is inserted; a host cell line transfected with the expression vector; or the antibody-drug conjugate (ADC) provided in the present invention.

An appropriate total daily amount used may be determined by an attending physician or veterinarian within the scope of sound medical judgment, and administration may be made once or several times. However, for the purposes of the present invention, a specific therapeutically effective amount for a particular patient is preferably applied differently depending on various factors, including type and degree of reaction to be achieved, the specific composition comprising the active ingredient, including whether other agents are used therewith as the case may be, the patient's age, body weight, general health status, sex, and diet, frequency of administration, route of administration, secretion rate of the composition comprising the active ingredient, duration of treatment, and drugs used simultaneously or in combination with the specific composition, and similar factors well known in the medical field.

Meanwhile, the method for preventing or treating a brain and nervous system disease may be, but is not limited to, a combination therapy that further comprises a step of administering a compound or substance having therapeutic activity against one or more diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, the dosage of the binding molecule or the antibody-drug conjugate may be, but is not limited to, about 0.0001 $\mu$g to 500 mg per kg of patient's body weight.

According to another embodiment of the present invention, there is provided a diagnostic composition for an immune-related disease comprising an agent for measuring the expression level of the Lrig-1 protein or its extracellular domain or a gene encoding the Lrig-1 protein present on the surface of a T cell using the binding molecule of the present invention.

The T cells of the present invention may be regulatory T cells.

The diagnostic composition of the present invention may comprise, for example, an agent that measures the expression level of the Lrig-1 protein or its extracellular domain or a gene encoding the Lrig-1 protein present on the surface of an activated regulatory T cell, that is, regulatory T cells with activated suppression of effector T cells, but is not limited thereto.

The diagnostic composition of the present invention may further comprise a protein, more than one selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells, and an agent for measuring the expression level of a gene encoding the same. As such, when the expression level of various proteins or genes encoding such is further measured on the surface of T cells, a significant synergistic effect can be achieved in diagnosing the target disease compared to measuring the Lrig-1 protein or its extracellular domain or gene encoding it alone.

Although the agent measuring the expression level of the Lrig-1 protein or its extracellular domains of the present invention, and the expression level of more than one protein selected from the group consisting of CD25, TIGIT, LAG3, CTLA-4, GITR, OX40, ICOS, PD-1, TIM-3, CCR4, FR4, CD15s, PI-16, and Lrig-1 protein are not particularly limited, it may comprise, for example, at least one selected from the group consisting of an antibody, oligopeptide, ligand, peptide nucleic acid (PNA), and aptamer that specifically binds to the protein.

The agent measuring the expression level of the gene encoding the Lrig-1 protein of the present invention (in other words, LAIR1) or the gene encoding the extracellular domain of the Lrig-1 protein, and a gene encoding more than one protein selected from the group consisting of CD25, TIGIT, LAG3, CTLA-4, GITR, OX40, ICOS, PD-1, TIM-3, CCR4, FR4, CD15s, PI-16, and Lrig-1 may comprise at least one selected from the group consisting of a primer, probe, LNA, and antisense nucleotides that specifically bind to the gene.

As used herein, the term "primer" refers to a fragment that recognizes a target gene sequence, and consists of a forward and reverse primer pair, but preferably provides analysis results with specificity and sensitivity. High specificity can be imparted when the primers amplify the target gene sequence containing complementary primary binding sites while not causing non-specific amplification because the nucleic acid sequence of the primer is inconsistent with the non-target sequence present in the sample.

As used herein, the term "probe" refers to a substance that can specifically bind to a target substance to be detected in a sample, and a substance that can specifically confirm the presence of a target substance in a sample through binding. Given probes are commonly used in the art, the type of probe is not limited, but preferably can be peptide nucleic acid (PNA), locked nucleic acid (LNA), peptide, polypeptide, protein, RNA, or DNA, with PNA being the most preferred. More specifically, the probe is a biomaterial that includes those derived from or similar to those from living organisms or those manufactured *in vitro,* for example, enzymes, proteins, antibodies, microorganisms, animal and plant cells and organs, nerve cells, DNA, and RNA. DNA may comprise cDNA, genomic DNA and oligonucleotides, while RNA may comprise genomic RNA, mRNA, oligonucleotides, while proteins may comprise antibodies, antigens, enzymes, peptides, and the like.

According to another embodiment of the present invention, there is provided a diagnostic composition for a brain and nervous system disease, comprising the binding molecule provided in the present invention.

In the present invention, the kit may be, but is not limited to, an RT-PCR kit, a DNA chip kit, an ELISA kit, a protein chip kit, a rapid kit, or a multiple reaction monitoring (MRM) kit.

The diagnostic kit of the present invention may further include one or more other component compositions, solutions, or devices suitable for assay methods. For example, the diagnostic kit of the present invention may further include essential elements required for performing reverse transcription polymerase reaction. The kit for reverse transcription polymerase reaction includes a pair of primers specific for a gene encoding a marker protein. The primer pair is a nucleotide having a sequence specific to a nucleic acid sequence of the gene, and may have a length of about 7 bp to 50 bp, or about 10 bp to 30 bp. The kit may also include a primer pair specific for a nucleic acid sequence of a control gene. In addition to those mentioned above, the kit for reverse transcription polymerase reaction may include test tubes or other suitable containers, reaction buffers (with varying pH and magnesium concentrations), deoxynucleotides (dNTPs), enzymes such as Taq-polymerase and reverse transcriptase, DNase and RNase inhibitors, DEPC-treated water, sterilized water, and the like.

In addition, the diagnostic kit of the present invention may include essential elements required for performing a DNA chip assay. The DNA chip kit may include a substrate to which a cDNA or oligonucleotide corresponding to a gene or a fragment thereof is attached; reagents, agents, and enzymes for preparing a fluorescence-labeled probe; and the like. The substrate may also contain a cDNA or oligonucleotide corresponding to a control gene or a fragment thereof.

In addition, the diagnostic kit of the present invention may include essential elements required for performing ELISA. The ELISA kit includes an antibody specific for the marker protein. The antibodies are antibodies with high specificity and affinity for the marker protein and little cross-reactivity with other proteins, and include monoclonal antibodies, polyclonal antibodies, or recombinant antibodies. The ELISA kit may also include an antibody specific for a control protein. In addition to those mentioned above, the ELISA kit may include reagents capable of detecting bound antibodies, for example, labeled secondary antibodies, chromophores, enzymes (which are, for example, conjugated with an antibody) and substrates thereof, and other substances capable of binding to antibodies.

According to another embodiment of the present invention, the present invention relates to a method for providing information on diagnosis of an immune-related disease comprising a step of measuring the expression level of Lrig-1 protein or extracellular domain thereof present on the surface of T cells in a biological sample isolated from a target subject using the binding molecule of the present invention, or the expression level of a gene encoding such.

The T cells of the present invention may be regulatory T cells.

The step of measuring the expression level of the present invention may be to further measure the expression levels of a protein, more than one selected from the group consisting of CD25, TIGIT, LAG3, CTLA-4, GITR, OX40, ICOS, PD-1, TIM-3, CCR4, FR4, CD15s, PI-16, and Lrig-1 proteins present on the surface of T cells, or the expression levels of a gene encoding the same. As such, when the expression level of various proteins or genes encoding such is further measured on the surface of T cells, a significant synergistic effect can be achieved in diagnosing the target disease compared to measuring the Lrig-1 protein or the gene encoding it alone.

In the present invention, the "target individual" refers to an individual for whom it is uncertain whether the disease has developed and who has a high probability of developing the disease.

In the present invention, the "biological sample" may refer to any material, biological fluid, tissue or cell obtained or derived from an individual, and can comprise, for example, whole blood, leukocytes, peripheral blood mononuclear cells, buffy coat, plasma, serum, sputum, tears, mucus, nasal washes, nasal aspirates, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid, but is not limited thereto.

The Lrig-1 protein or extracellular domain thereof of the present invention may be expressed on the cell surface of T cells, in particular, regulatory T cells, for example, activated regulatory T cells, that is the cell surface of activated regulatory T cells with activated suppressive ability of effector T cells.

In the present invention, examples of the method for measuring or comparatively analyzing the expression level of the Lrig-1 protein, extracellular domain thereof, and more than one protein selected from the group consisting of CD25, TIGIT, LAG3, CTLA-4, GITR, OX40, ICOS, PD-1, TIM-3, CCR4, FR4, CD15s, PI-16 and Lrig-1 include, but are not limited to, protein chip assay, immunoassay, ligand binding assay, matrix assisted laser desorption/ionization time of flight mass spectrometry (MALDI-TOF), sulface enhanced laser desorption/ionization time of flight mass spectrometry (SELDI-TOF), radioimmunoassay, radial immunodiffusion, ouchterlony immunodiffusion, rocket immunoelectrophoresis, immunohistochemical staining, complement fixation assay, two-dimensional electrophoresis, liquid chromatography-mass spectrometry (LC-MS), liquid chromatography-mass spectrometry/mass spectrometry (LC-MS/MS), Western blotting, and enzyme linked immunosorbent assay (ELISA).

The analysis method for measuring the expression level of a gene in order to confirm the presence and expression level of genes encoding the Lrig-1 protein or its extracellular domain, and more than one protein selected from the group consisting of CD25, TIGIT, LAG3, CTLA-4, GITR, OX40, ICOS, PD-1, TIM-3, CCR4, FR4, CD15s, PI-16 and Lrig-1 may be, but is not limited to, reverse transcription polymerase reaction (RT-PCR), competitive RT-PCR, real-time RT-PCR, RNase protection assay (RPA), Northern blotting or DNA chip.

In the present invention, the method may comprise a step of predicting that the target individual has a high probability of developing an immune-related disease in a case where the expression level of Lrig-1 protein or its extracellular domain, or the gene encoding it measured in the biological sample of the target subject is decreased compared to the normal control.

In the present invention, the method may comprise a step of predicting that the target individual has a high probability of developing an immune-related disease in a case where the expression level of more than one protein selected from the group consisting of CD25, TIGIT, LAG3, CTLA-4, GITR, OX40, ICOS, PD-1, TIM-3, CCR4, FR4, CD15s, PI-16 and Lrig-1 proteins, or where the expression level of the genes encoding such proteins is changed (increased or decreased) compared to the normal control.

In the present invention, the method may comprise a step of predicting that the target individual has a high probability of developing an immune-related disease in a case where the expression level of Lrig-1 protein or the gene encoding it is decreased compared to a normal control.

Furthermore, in the case of predicting or diagnosing that an immune-related disease is highly likely to occur by measuring the expression level of Lrig-1 protein or its extracellular domain or the gene encoding it, the method may further comprise a step of administering a drug for the disease to the individual.

According to one embodiment of the present invention, there is provided a binding molecule comprising a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

According to one embodiment of the present invention, there is provided a binding molecule comprising a heavy chain variable region comprising a heavy chain CDR1 encoded by the nucleotide sequence represented by SEQ ID NO: 13, a heavy chain CDR2 encoded by the nucleotide sequence represented by SEQ ID NO: 14, and a heavy chain CDR3 encoded by the nucleotide sequence represented by SEQ ID NO: 15, and a light chain variable region comprising a light chain CDR1 encoded by the nucleotide sequence represented by SEQ ID NO: 16, a light chain CDR2 encoded by the nucleotide sequence represented by SEQ ID NO: 17, and a light chain CDR3 encoded by the nucleotide sequence represented by SEQ ID NO: 18.

According to one embodiment of the present invention, there is provided a binding molecule comprising a heavy chain variable region consisting of the amino acid sequence represented by SEQ ID NO: 11, and a light chain variable region consisting of the amino acid sequence represented by SEQ ID NO: 12.

According to one embodiment of the present invention, there is provided a binding molecule comprising a heavy chain variable region encoded by the nucleotide sequence represented by SEQ ID NO: 19, and a light chain variable region encoded by the nucleotide sequence represented by SEQ ID NO: 20.

According to one embodiment of the present invention, there is provided a binding molecule that further comprises a fragment crystallization region (Fc region) or a constant region.

According to one embodiment of the present invention, there is provided a binding molecule wherein the Fc region is an Fc region of an IgA, IgD, IgE, IgM, IgG1, IgG2, IgG3 or IgG4, or a hybrid Fc.

According to one embodiment of the present invention, there is provided a binding molecule that further comprises a heavy chain constant region consisting of an amino acid sequence selected from the group consisting of SEQ ID NO: 21, 23, 24, 27, 28, 29, 30 and 32.

According to one embodiment of the present invention, there is provided a binding molecule that further comprises a light chain constant region consisting of an amino acid sequence selected from the group consisting of SEQ ID NO: 22, 25, 26, and 31.

According to one embodiment of the present invention, there is provided a binding molecule that further comprises a heavy chain constant region encoded by the nucleotide sequence represented by SEQ ID NO: 33 or 35, and a light chain constant region encoded by the nucleotide sequence represented by SEQ ID NO: 34 or 36.

According to one embodiment of the present invention, there is provided a binding molecule that comprises a heavy chain consisting of the amino acid sequence represented by SEQ ID NO: 37 or 38, and a light chain consisting of the amino acid sequence represented by SEQ ID NO: 39 or 40.

According to one embodiment of the present invention, there is provided a binding molecule that comprises a heavy chain consisting of the amino acid sequence represented by SEQ ID NO: 37, and a light chain consisting of the amino acid sequence represented by SEQ ID NO: 39.

According to one embodiment of the present invention, there is provided a binding molecule that comprises a heavy chain consisting of the amino acid sequence represented by SEQ ID NO: 38 and a light chain consisting of the amino acid sequence represented by SEQ ID NO: 40.

According to one embodiment of the present invention, there is provided a binding molecule that comprises a heavy chain encoded by the nucleotide sequence represented by SEQ ID NO: 41 or 43, and a light chain encoded by the nucleotide sequence represented by SEQ ID NO: 42 or 44.

According to one embodiment of the present invention, there is provided a binding molecule that comprises a heavy chain encoded by the nucleotide sequence represented by SEQ ID NO: 41, and a light chain encoded by the nucleotide sequence represented by SEQ ID NO: 42.

According to one embodiment of the present invention, there is provided a binding molecule that comprises a heavy chain encoded by the nucleotide sequence represented by SEQ ID NO: 43 and a light chain encoded by the nucleotide sequence represented by SEQ ID NO: 44.

According to one embodiment of the present invention, the binding molecule provides a binding molecule that is an antibody or a fragment thereof.

According to one embodiment of the present invention, there is provided a binding fragment wherein the antibody is a chimeric antibody, a humanized antibody, a bivalent, bispecific molecule, a minibody, a domain antibody, a bispecific antibody, an antibody mimic, an unibody, a diabody, a triabody, a tetrabody, or fragments thereof.

According to one embodiment of the present invention, a nucleic acid molecule encoding the binding molecule is provided.

According to one embodiment of the present invention, an expression vector into which the nucleic acid molecule is inserted is provided.

According to one embodiment of the present invention, a host cell line transfected with the expression vector is provided.

According to one embodiment of the present invention, there is provided an antibody-drug conjugate (ADC) that comprises an antibody that comprises a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10, and a drug.

According to one embodiment of the present invention, there is provided a pharmaceutical composition for preventing or treating a brain and nervous system disease comprising the binding molecule of the present invention as an active ingredient.

According to one embodiment of the present invention, there is provided a pharmaceutical composition wherein the brain and nervous system disease is a neurodegenerative disease or a neuroinflammatory disease.

According to one embodiment of the present invention, there is provided a pharmaceutical composition wherein the neurodegenerative disease or neuroinflammatory disease is at least one selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

According to one embodiment of the present invention, in a chimeric antigen receptor (CAR) comprising an antigen-specific binding domain, a linking domain and a CD3 zeta (ζ) signaling domain, there is provided a chimeric antigen receptor wherein the specific binding domain is a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

According to one embodiment of the present invention, there is provided a pharmaceutical composition for preventing or treating a brain and nervous system disease comprising the chimeric antigen receptor as an active ingredient.

According to one embodiment of the present invention, there is provided a diagnostic composition for a brain and nervous system disease comprising the binding molecule of the present invention.

According to one embodiment of the present invention, there is provided a diagnostic composition for a brain and nervous system disease comprising the composition of the present invention.

According to one embodiment of the present invention, there is provided a method for providing information for diagnosing a brain and nervous system disease comprising the step of measuring the expression level of Lrig-1 protein on the surface of T cells in a biological sample isolated from a target subject using the binding molecule of the present invention.

According to another embodiment of the present invention, there is provided a method for preventing or treating a brain and nervous system disease characterized by a binding molecule comprising a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

According to one embodiment of the present invention, the binding molecule is an antibody or a fragment thereof, and there is provided a method for preventing or treating a brain and nervous system disease wherein the antibody is characteristically a chimeric antibody, a humanized antibody, a bivalent, a bispecific molecule, a minibody, a domain antibody, a bispecific antibody, an antibody mimic, an unibody, a diabody, a triabody, a tetrabody, or fragments thereof.

According to another embodiment of the present invention, there is provided a method for preventing or treating a brain and nervous system disease characterized by an antibody-drug conjugate comprising an antibody that comprises a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10, and a drug.

According to one embodiment of the present invention, in a chimeric antigen receptor (CAR) comprising an antigen-specific binding domain, a linking domain and a CD3 zeta (ζ) signaling domain, there is provided a method for preventing or treating a brain and nervous system disease characterized by a chimera antigen receptor, wherein the specific binding domain is a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

Advantageous Effects of Invention

The binding molecule provided in the present invention can specifically prevent, ameliorate, or treat brain and nervous system diseases such as various neurodegenerative or neuroinflammatory diseases.

Brief Description of Drawings

FIG. 1 illustrates a structure of the Lrig-1 protein according to an embodiment of the present invention.

FIG. 2 illustrates a structure of the Lrig-1 protein according to an embodiment of the present invention.

FIG. 3 illustrates an expression level of Lrig-1 mRNA according to an embodiment of the present invention.

FIG. 4 illustrates an expression level of Lrig-1 mRNA according to an embodiment of the present invention.

FIG. 5 illustrates an expression level of Lrig-1 mRNA according to an embodiment of the present invention.

FIG. 6 illustrates expression levels of Lrig-1, Lrig-2, and Lrig-3 mRNAs according to an embodiment of the present invention.

FIG. 7 illustrates results obtained by comparing expression levels of Lrig-1 protein in regulatory T cells and non-regulated T cells according to an embodiment of the present invention.

FIG. 8 illustrates expression level of the Lrig-1 protein on the surface of regulatory T cells according to an embodiment of the present invention.

FIG. 9 illustrates an experimental design for identifying therapeutic effects of monoclonal antibodies on Alzheimer's, according to an embodiment of the present invention.

FIG. 10 illustrates a photograph of results obtained with a fluorescence microscope by staining the brain cortex and hippocampal tissue of the mouse with Thioflavin S (ThS) obtained after each treatment of Alzheimer's-induced mice with the monoclonal antibody according to an embodiment of the present invention.

FIG. 11 illustrates a graph showing the results of calculating the ThS+ area ratio (%) relative to normal mice after staining the brain cortex and hippocampal tissue of the mouse with Thioflavin S (ThS) obtained after each treatment of Alzheimer's-induced mice with the monoclonal antibody according to an embodiment of the present invention.

FIG. 12 illustrates a graph showing changes in spontaneous alternation (%) values as a result of Y-maze test after each treatment of Alzheimer's-induced mice with the monoclonal antibody according to an embodiment of the present invention.

FIG. 13 illustrates a graph showing changes in preference values as a result of a novel object recognition experiment after each treatment of Alzheimer's-induced mice with the monoclonal antibody according to an embodiment of the present invention.

FIG. 14 illustrates a graph showing changes in the time taken to reach the position of the platform as a result of the water maze experiment after each treatment of Alzheimer's-induced mice with the monoclonal antibody according to an embodiment of the present invention.

FIG. 15 illustrates a graph showing changes in the number of times the Alzheimer's-induced mice passed the target as a result of the water maze experiment after each treatment of Alzheimer's-induced mice with the monoclonal antibody according to an embodiment of the present invention.

FIG. 16 illustrates a graph showing calculated changes in the time taken to reach the position of the platform during the water maze experiment after each treatment of Alzheimer's-induced mice with the monoclonal antibody according to an embodiment of the present invention.

FIG. 17 illustrates an experimental design of Alzheimer's-induced 5xFAD mice and 6xTg mice for identifying therapeutic effects of monoclonal antibodies on Alzheimer's, according to an embodiment of the present invention.

FIG. 18 illustrates a graph showing changes in spontaneous alternation (%) values as a result of Y-maze test after each treatment of Alzheimer's-induced 5xFAD mice and 6xTg mice with the monoclonal antibody according to an embodiment of the present invention.

FIG. 19 illustrates a graph showing changes in preference values as a result of a novel object recognition experiment after each treatment of Alzheimer's-induced 6xTg mice with the monoclonal antibody according to an embodiment of the present invention.

FIG. 20 illustrate a graph showing changes in retention time as a result of a passive avoidance experiment after each treatment of Alzheimer's-induced 5xFAD mice and 6xTg mice with the monoclonal antibody according to an embodiment of the present invention.

Best Description for Implementing the Invention

According to one embodiment of the present invention, there is provided a binding molecule comprising a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7, and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

Detailed Description of Invention

Hereinafter, the present invention will be described in more detail by way of examples. These examples are only for describing the present invention in more detail, and it will be apparent to those skilled in the art that according to the gist of the present invention, the scope of the present invention is not limited by these examples.

Examples

**[Preparation Example] T cell subset cell culture**

In order to identify whether the Lrig-1 protein is expressed only in regulatory T cells (Treg), the subsets of T cells, Th0, Th1, Th2, Th17, and iTreg, were prepared. The iTreg refers to cells whose differentiation has been artificially induced in a medium containing the following composition, unlike nTreg which has been naturally isolated.

The subsets of the T cells were induced to differentiate into respective cells by first isolating naive T cells obtained from the spleen of mice, causing RPMI1640 (Invitrogen Gibco, Grand Island, NY) nutrient medium that contains 10% fetal bovine serum (FBS; HyClone, Logan, UT) to further contain the respective ingredients of Table 1 below, and performing 72-hour incubation in an incubator at 37°C, 5% $CO_2$.

[Table 1]

| Differentiated cell | Composition |
|---|---|
| Th0 | anti-CD3, anti-CD28 |
| Th1 | IL-12, anti-IL-4 antibody |
| Th2 | IL-4, anti-IFNβ |
| Th17 | IL-6, TGFP, anti-IFNβ, anti-IL-4 |
| iTreg | IL-2, TGFβ |

**[Example 1] Structural analysis of Lrig-1**

A three-dimensional steric structure of the extracellular domain of the Lrig-1 protein was predicted to produce antibodies specific for the Lrig-1 protein, a surface protein of regulatory T cells.

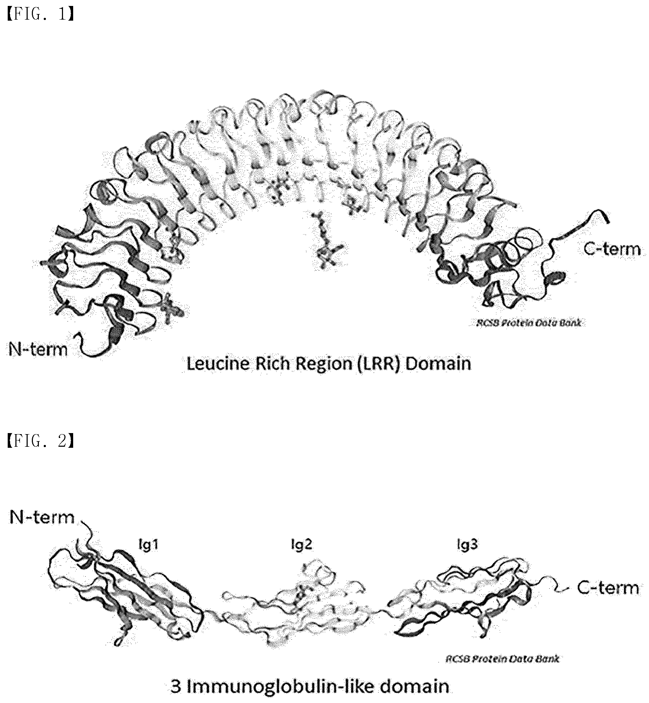

First, in order to predict base sequences of epitopes (epitopes), tools of Uniprot (http://www.uniprot.org) and RCSB Protein Data Bank (http://www.rcsb.org/pdb) were used to predict a three-dimensional steric structure of the extracellular domain (ECD) of the Lrig-1 protein so that the structure of ECD is identified. Then, the results are illustrated in FIGS. 1 and 2.

As illustrated in FIG. 1, a total of 15 leucine-rich regions of LRR1 to LRR15 existed in the Lrig-LRR domain (amino acid sequence at positions 41 to 494) in the extracellular domain of the Lrig-1 protein. Each of the LRR domains is composed of 23 to 27 amino acids, with 3 to 5 leucine being present.

In addition, as illustrated in FIG. 2, three immunoglobulin-like domains exist in amino acid sequences at positions 494 to 781 of the Lrig-1 protein in the extracellular domain of the Lrig-1 protein.

### [Example 2] Prediction of Lrig-1 epitope amino acid sequence

Prediction of the above base sequence was performed using Ellipro server (http://tools.iedb.org/ellipro/) which is an epitope prediction software based on a structure of the Lrig-1 protein. The Ellipro search engine was used because it corresponds to a search engine known to be the most reliable among the existing algorithms for predicting an epitope.

The extracellular domain analyzed in Example 1 was entered into the epitope prediction software, and a total of 22 contiguous epitope amino acid sequences were predicted, and a total of 8 discontinuous epitope amino acid sequences were predicted.

### [Production Examples 1 and 2] Production of monoclonal antibodies specific to Lrig-1 protein

Antibodies specific for the Lrig-1 protein according to the present invention were produced. The present antibodies were not produced by specifying a certain epitope, but were produced as antibodies capable of binding to any site on the Lrig-1 protein.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which a DNA sequence of the Lrig-1 protein is inserted. The thus produced pcDNA into which SEQ ID NO: 2 is inserted was introduced, through transfection, into T cells, so that the Lrig-1 protein is allowed to be expressed on the surface of the T cells.

Light and heavy chain amino acid sequences capable of binding to Lrig-1 expressed on the cell surface were selected from the Human scFv library.

The selected heavy and light chain amino acid sequences were fused with the heavy chain constant region represented by SEQ ID NO: 24 or 30 and the light chain constant region represented by SEQ ID NO: 26 or 31, respectively, to produce monoclonal antibodies. The sequences of the monoclonal antibodies are shown in Table 2 below.

[Table 2]

| Classification | Clone | Location | Amino acid sequence | Sequence information |
|---|---|---|---|---|
| Production | GTC310-01 mouse | Heavy | EVQLLESGGGLVQPGGSLRLSCAASG FTFS NYAMS WVRQAPGKGLEWVS | SEQ ID NO: |
| Example 1 | antibody | chain | VISHGGGSTYYADSVKG RFTISRDNSKNTLYLQMNSLRAEDTA VYYCAR VISNCHLGVCYYSNGMDV WGQGTLVTVSSAKTTAPSVYPLAPVC GDTTGSSVTLGCLVKGYFPEPVTLTW NSGSLSSGVHTFPAVLQSDLYTLSSSV TVTSSTWPSQSITCNVAHPASSTKVD KKIEPRGPTIKPCPPCKCPAPNLLGGPS VFIFPPKIKDVLMISLSPIVTCVVVDVS EDDPDVQISWFVNNVEVHTAQTQTH REDYNSTLRVVSALPIQHQDWMSGK EFKCKVNNKDLPAPIERTISKPKGSVR APQVYVLPPPEEEMTKKQVTLTCMV TDFMPEDIYVEWTNNGKTELNYKNT EPVLDSDGSYFMYSKLRVEKKNWVE RNSYSCSVVHEGLHNHHTTKSFSRTP GK | 37 |
| | | Light chain | QLVLTQPPSVSAAPGQRVSISC SGSSSNIGDNYVS WYQQVPGSAPKLLIY DNNKRPS GIPDRFSASKSGTSATLGITGLQTGDE ADYYCAT WDGSLSAGRV FGGGTKLTVL RADAAPTVSIFPPSSEQLTSGGASVVC FLNNFYPKDINVKWKIDGSERQNGVL NSWTDQDSKDSTYSMSSTLTLTKDEY ERHNSYTCEATHKTSTSPIVKSFNRNE C | SEQ ID NO: 39 |

| Classification | Clone | Location | Amino acid sequence | Sequence information |
|---|---|---|---|---|
| Production Example 2 | GTC310-01 humanized antibody | Heavy chain | EVQLLESGGGLVQPGGSLRLSCAASGFTFS NYAMS WVRQAPGKGLEWVSVISHGGGSTYYADSVKGRFTISRDNSKNTLYLQMNSLRAEDTAVYYCAR VISNCHLGVCYYSNGMDVWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK | SEQ ID NO: 38 |
| | | Light chain | QLVLTQPPSVSAAPGQRVSISCSGSSSNIGDNYVSWYQQVPGSAPKLLIY DNNKRPSGIPDRFSASKSGTSATLGITGLQTGDEADYYCAT WDGSLSAGRVFGGGTKLTVLRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | SEQ ID NO: 40 |

EP 4 286 412 A1

21

**[Example 3] Identification of specific expression of Lrig-1 mRNA in regulatory T cells**

Verification was made of whether the Lrig-1 protein can act as a biomarker specific for regulatory T cells.

For the verification, CD4+ T cells were isolated using magnet-activated cell sorting (MACS), through CD4 beads, from the spleen of mice. Subsequently, regulatory T (CD4+CD25+ T) cells and non-regulatory T (CD4+CD25- T) cells were isolated with a fluorescence-activated cell sorter (FACS) using a CD25 antibody. For the respective cells and the cells differentiated in Preparation Example 1, mRNA was extracted using Trizol, and gDNA was removed from genomic RNA using gDNA extraction kit (Qiagen) according to the protocol provided by the manufacturer. The gDNA-removed mRNA was synthesized into cDNA through the BDsprint cDNA Synthesis Kit (Clonetech).

Real-time polymerase chain reaction (RT PCR) was performed to quantitatively identify an expression level of Lrig-1 mRNA in the cDNA.

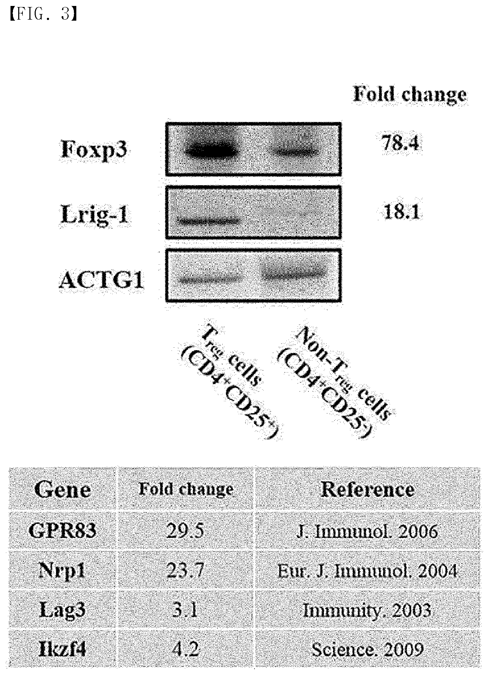

The real-time polymerase chain reaction was performed with primers shown in Table 3 below using SYBR Green (Molecular Probes) by the protocol provided by the manufacturer under conditions of 40 cycles consisting of 95°C for 3 minutes, 61°C for 15 seconds, 72°C for 30 seconds, a relative gene expression level was calculated using the $\Delta$CT method, and normalized using HPRT. The results are illustrated in FIGS. 3 to 6.

[Table 3]

| Primer | Sequence | Sequence Information |
|---|---|---|
| Mouse Lrig-1 | Forward 5' - GAC GGA ATT CAG TGA GGA GAA CCT - 3' | SEQ ID NO: 45 |
| | Reverse 5' - CAA CTG GTA GTG GCA GCT TGT AGG - 3' | SEQ ID NO: 49 |
| Mouse Lrig-2 | forward 5' - TCA CAA GGA ACA TTG TCT GAA CCA- 3' | SEQ ID NO: 47 |
| | reverse 5' - GCC TGA TCT AAC ACA TCC TCC TCA- 3' | SEQ ID NO: 48 |
| Mouse Lrig-3 | forward 5' - CAG CAC CTT GAG CTG AAC AGA AAC - 3' | SEQ ID NO: 49 |
| | reverse 5' - CCA GCC TTT GGT AAT CTC GGT TAG - 3' | SEQ ID NO: 50 |
| Mouse | forward 5' - CTT TCA CCT ATC CCA CCC TTA TCC - 3' | SEQ ID NO: 51 |
| FOXP3 | reverse 5' - ATT CAT CTA CGG TCC ACA CTG CTC - 3' | SEQ ID NO: 52 |
| ACTG1 | forward 5' - GGC GTC ATG GTG GGC ATG GG - 3' | SEQ ID NO: 43 |
| | reverse 5' - ATG GCG TGG GGA AGG GCG TA - 3' | SEQ ID NO: 54 |

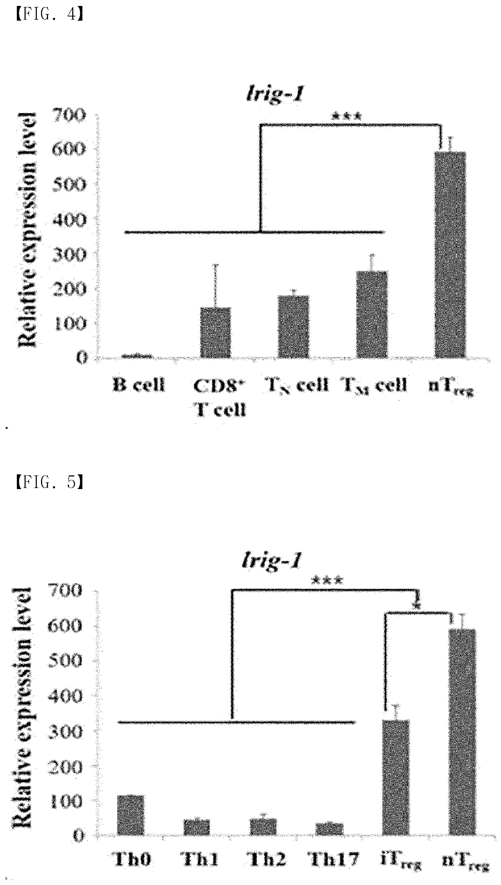

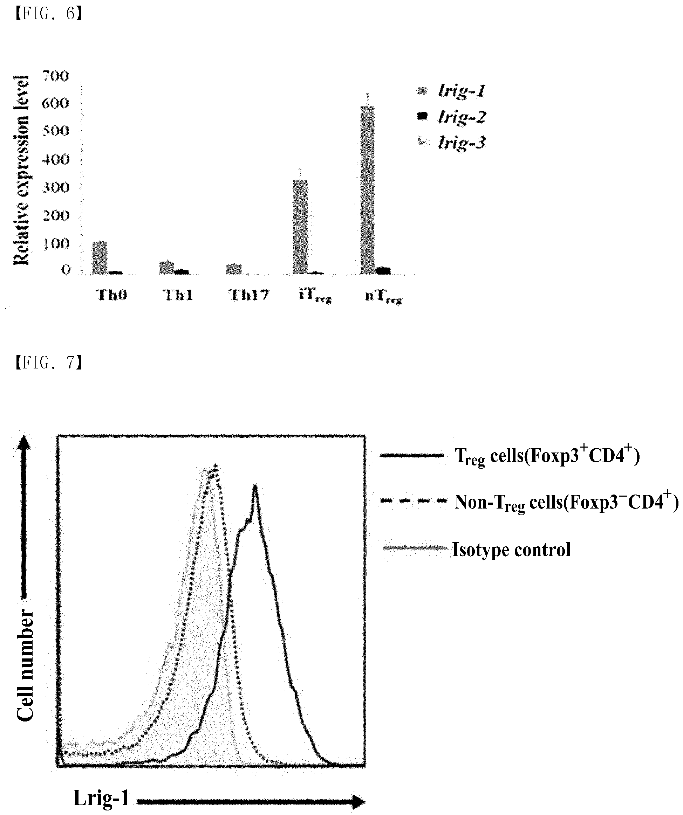

As illustrated in FIG. 3, it can be seen that the expression of Lrig-1 in regulatory T (CD4+CD25+ T) cells is 18.1 times higher than non-regulatory T (CD4+CD25- T) cells. This was about 10 times higher expression level than Lag3 and Ikzf4, which are previously known markers for regulatory T cells. In addition, as illustrated in FIGS. 4 and 5, the expression of Lrig-1 mRNA was remarkably high in regulatory T cells as compared with other types of immune cells, and in particular, was remarkably high in naturally isolated regulatory T cells (nTreg) as compared with induced regulatory T cells (iTreg cells). In addition, as illustrated in FIG. 6, expression of Lrig-1 was the highest among Lrig-1, Lrig-2, and Lrig-3 which correspond to the Lrig family.

From the above results, it can be seen that the Lrig-1 protein according to the present invention is specifically expressed in regulatory T cells, in particular, naturally-occurring regulatory T cells.

**[Example 4] Identification of specific expression of Lrig-1 protein in regulatory T cells**

It was identified whether the Lrig-1 protein expressed from Lrig-1 mRNA is specifically expressed only in regulatory T cells.

Using FOXP3-RFP-knocked-in mice, the FOXP3-RFP obtained by coupling red fluorescence protein (RFP) to FOXP3 promoter, a transcription factor specific for regulatory T cells, CD4+ T cells were isolated using magnet-activated cell sorting (MACS), through CD4 beads, from the spleen of the mice. Subsequently, using RFP protein, regulatory T (CD4+RFP+ T) cells and non-regulatory T (CD4+RFP- T) cells were obtained by performing isolation through a fluorescence-activated cell sorter (FACS). The respective cells were stained with the purchased Lrig-1 antibody and a negative control was stained with an isotype-matched control antibody, to measure an expression level of Lrig-1 with the fluorescence-activated cell sorter. The results are illustrated in FIG. 7.

As illustrated in FIG. 7, the non-regulatory T cells indicated by a dotted line showed almost the same expression level of Lrig-1 as the negative control, whereas there were a large number of cells with high expression level of Lrig-1 in the regulatory T cells.

From the above results, it can be seen that the Lrig-1 protein according to the present invention is specifically expressed in regulatory T cells.

## [Example 5] Identification of specific expression of Lrig-1 protein on surface of regulatory T cells

From the viewpoint that in order to be a target of cell therapy, the Lrig-1 protein must be expressed on the surface of regulatory T cells, which in turn allows a more effective target therapy, it was identified whether the Lrig-1 protein is expressed on the surface of the regulatory T cells.

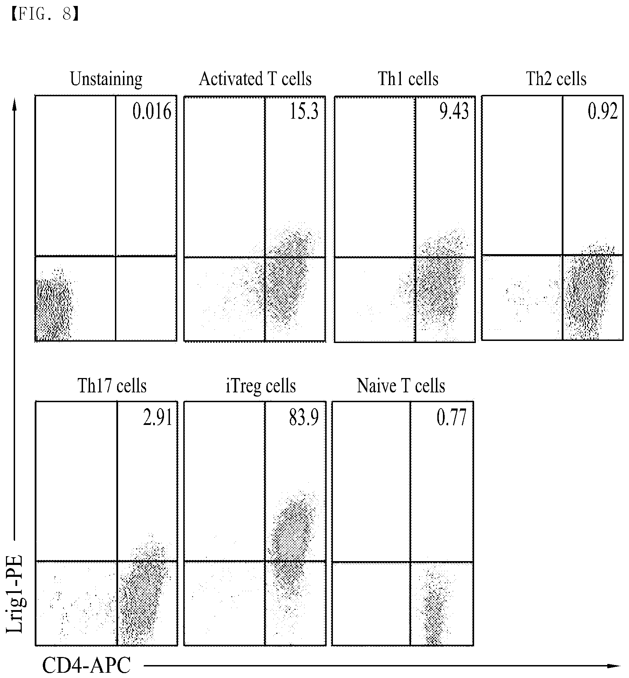

The respective differentiated T cell subsets of Preparation Example 1 were stained with anti-CD4-APC and anti-Lrig-1-PE antibodies, and expression levels of Lrig-1 were measured at the respective cell surfaces using a fluorescence-activated cell sorter (FACS). The results are illustrated in FIG. 8.

As illustrated in FIG. 8, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is, in particular, expressed at a higher level on the surface of the Treg cells.

## [Example 6] Evaluation of therapeutic ability of antibody according to present invention against Alzheimer's disease - 5xFAD mice

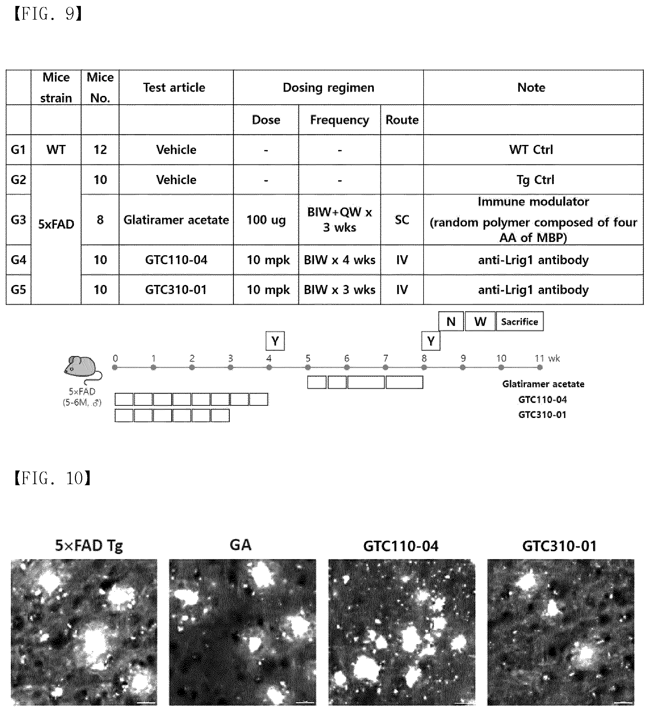

In order to evaluate the therapeutic ability of the antibody according to the present invention against Alzheimer's disease, groups were designed as shown in FIG. 9. Specifically, 5- to 6-month-old male 5xFAD mice with induced Alzheimer's disease were intravenously injected with GTC310-01 mouse antibody at an amount of 10mpk each for 3 weeks. However, glatiramer acetate (GA, Copaxone®) was subcutaneously injected at an amount of 100 $\mu$g for 3 weeks, or the H6 antibody (GTC110-04) shown in Table 4 was intravenously injected in an amount of 10mpk for 4 weeks as a positive control.

[Table 4]

| Clone | Location | Amino Acid Sequence |
|---|---|---|
| H6 antibody (GTC110-04) | Heavy chain | EVQLLESGGGLVQPGGSLRLSCAASGFTFSNYAMSWVRQAPGKG LEWVSVISHGGGSTYYADSVKGRFTISRDNSKNTLYLQMNSLRA EDTAVYYCARVISNCHLGVCYYSNGMDVWGQGTLVTVSSTTAPS VYPLAPVCGDTTGSSVTLGCLVKGYFPEPVTLTWNSGSLSSGVH TFPAVLQSDLYTLSSSVTVTSSTWPSQSITCNVAHPASSTKVDK KIEPRGPTIKPCPPCKCPAPNLLGGPSVFIFPPKIKDVLMISLS PIVTCVVVDVSEDDPDVQISWFVNNVEVHTAQTQTHREDYNSTL RVVSALPIQHQDWMSGKEFKCKVNNKDLPAPIERTISKPKGSVR APQVYVLPPPEEEMTKKQVTLTCMVTDFMPEDIYVEWTNNGKTE LNYKNTEPVLDSDGSYFMYSKLRVEKKNWVERNSYSCSVVHEGL HNHHTTKSFSRTPGK |
| | Light chain | QSVLTQPPSASGTPGQRVTISCSGSSSNIGNNDVYWYQQLPGTA PKLLIYSDSQRPSGVPDRFSGSKSGTSASLAISGLRSEDEADYY CGTWDYSLSGYVFGGGTKLTVLRTVAAPTVSIFPPSSEQLTSGG ASVVCFLNNFYPKDINVKWKIDGSERQNGVLNSWTDQDSKDSTY SMSSTLTLTKDEYERHNSYTCEATHKTSTSPIVKSFNRNEC |

**1. Amyloid-β plaque reducing effect**

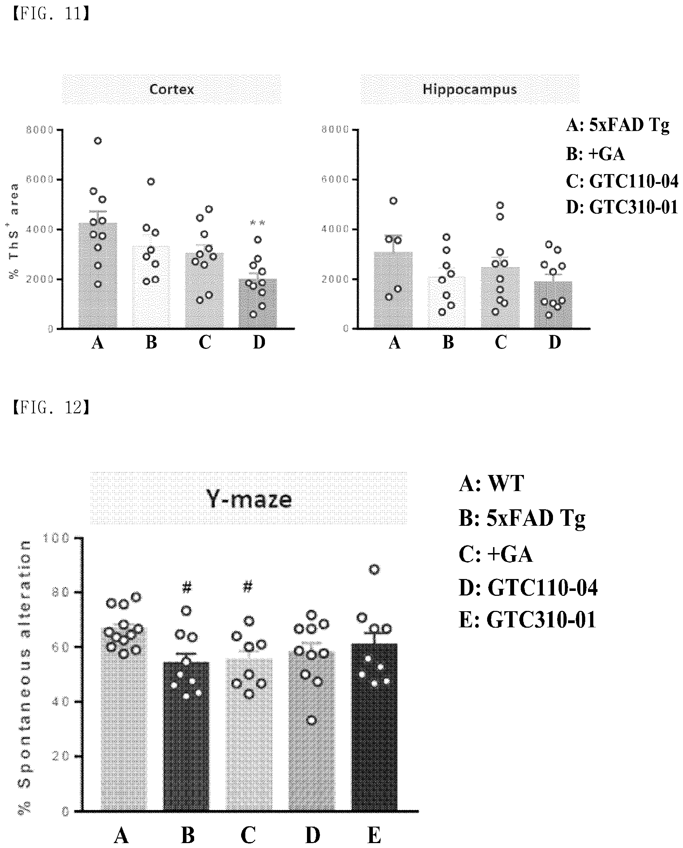

For immunohistological analysis, the above-treated mice were euthanized, and the cortex and hippocampus tissues were obtained and fixed in 4% paraformaldehyde (pH 7.4) for 16 hours. The fixed cortex and hippocampus tissues were immersed in 30% sucrose for cryoprotection and sliced to a thickness of 35 $\mu$m. To visualize Aβ plauqes, the sliced brain sections were stained with thioflavin S (ThS) for 7 minutes. Thioflavin S was purchased from Sigma-Aldrich (catalog number T-1892). Thioflavin S (ThS) at 500 $\mu$M was dissolved in 50% ethanol. After successive washes with 100%, 95% and 70% ethanol, the sections were transferred to PBS. Relatively thick free-floating brain slices were incubated with goat anti-GFAP antibody for 7 days at 4°C for immunofluorescence. Then, the sections were incubated overnight at 4°C with Alexa Fluor 594-conjugated donkey anti-goat IgG (1:200, Abeam, ab150132). Nuclear counterstaining was performed using 4'6'-damiino-2-phenylindole dihydrochloride hydrate (DAPI, 1 mg/mL, 1:2000, Sigma). Images were taken with a Leica DM2500 fluorescence microscope, and the results are shown in FIG. 10. Additionally, the ThS+ area ratio (%) relative to normal mice was calculated using the ImageJ software program, and the results are shown in FIG. 11.

As shown in FIGS. 10 and 11, although the ThS+ area significantly increased in Alzheimer's-induced mice, when the GTC310-01 antibody according to the present invention was administered, the ThS+ area was significantly reduced, especially in the cortex, thus showing that the effect of reducing amyloid-β plaques was excellent.

**2. Y-maze test**

For the Y-maze test, a Y-shaped maze was used which had three identical arms having a length of 40 cm (with a 15-cm high wall) at an angle of 120 degrees from each other. This experiment was a behavioral experiment using the rodent's instinctive exploring habit and was based on the fact that there is a high possibility of exploring a new area. The higher the degree that the test animal remembered the arm it had just explored and tried not to enter the same arm, the higher the animal's memory level. An exploring time of 8 minutes was given to each individual, and the final results were expressed as spontaneous alteration (%) values in FIG. 12. The spontaneous alteration (%) value was calculated by Expression 1. Here, behavioral patterns were analyzed using SMART VIDEO TRACKING Software (Panlab, USA).

[Equation 1]

$$\text{Spontaneous alteration } (\%) = \frac{number\ of\ triplet}{total\ arm\ entry - 2}$$

As shown in FIG. 12, in this experiment for measuring the relative frequency at which the test animal perceives the surrounding clues and sequentially enters the maze, it was found that in the case where the mice with induced Alzheimer's disease were administered with the GTC310-01 antibody according to the present invention, the mice had a spontaneous alteration value at a level similar to that of a normal control.

**3. Novel Object Recognition**

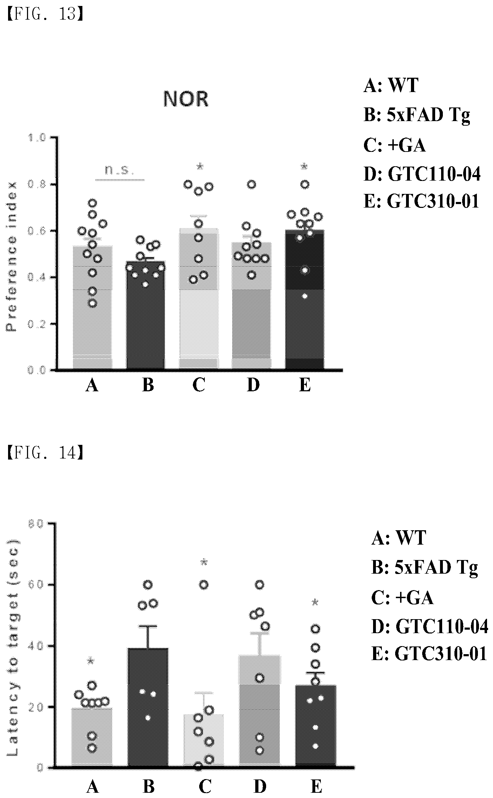

The novel object recognition test was performed to evaluate memory using two different objects in a 40 cm × 40 cm acrylic cage. After causing the test animal to acclimatize to the inside of the acrylic cage, two objects were placed at certain locations and the animal was allowed to perceive the objects freely. Then, the time for exploring each object was measured. 24-hour delay was given to each individual, and only one object was changed to another at the same location. Here, in a case where the animal perceives the changed object as a novel object and spends a longer exploring time, it may be determined that the animal has better memory. In a case where the animal does not remember the objects that it explored 24 hours before, the animal fails to distinguish between the novel object and the old object, and thus there is a possibility that the animal explores equally both objects. The animal was allowed to explore freely for a total of 10 minutes, and the result was expressed as a preference index (which equals novel object exploring time/total exploring time) in FIG. 13. Here, the analysis was performed using SMART VIDEO TRACKING Software (Panlab, USA).

As shown in FIG. 13, the preference index for a new object was lower in the mice with induced Alzheimer's disease compared with the normal control. However, when the GTC310-01 antibody according to the present invention was administered, the preference value was higher than that of the normal control.

**4. Water maze test**

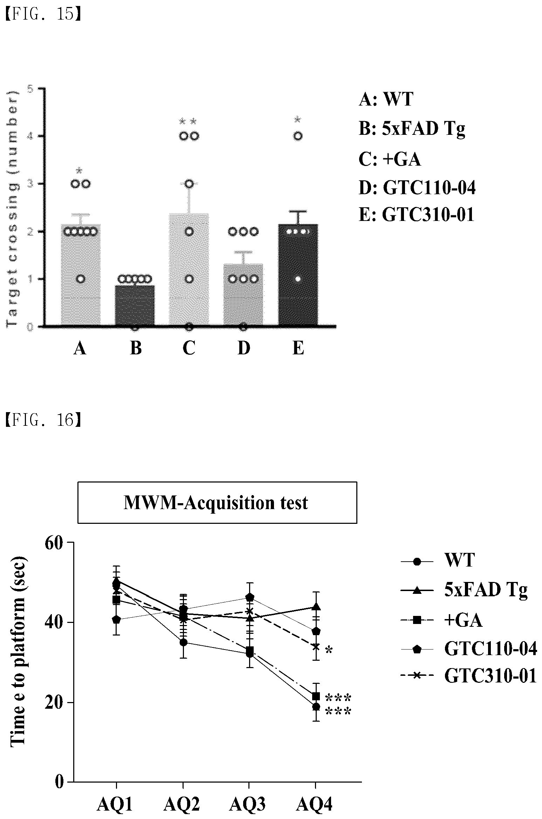

The water maze test was conducted based on the method devised by Morris. A stainless-steel pool (90 cm in diameter, 50 cm in height) was filled with water (22 $\pm$ 1°C) so that the height of the water surface was 30 cm. A hidden platform (5 cm in diameter) was placed 1 cm below the water surface. On day 1 of evaluation, 3 to 4 training sessions were allowed. A total swimming time per individual was set to 60 seconds; and the individual, who found the platform within 60 seconds, was allowed to stay on the platform for 10 seconds to induce memory. The individual, who was unable to find the platform even after 60 seconds, was manually guided to the platform and allowed to stay on the platform for 10 seconds, wherein the time to escape was set to 60 seconds. On day 6 after the training, a probe test was conducted to calculate spatial perception (time taken to reach the location of the platform (latency to target, sec)) and the number of times it crossed the target (target crossing numbers, number), the results of which are shown in FIGS. 14 and 15, and an acquisition test was conducted to calculate the time taken to reach the platform (time to platform, sec), the result of which is shown in FIG. 16. The analysis was performed using SMART VIDEO TRACKING Software (Panlab, USA). In interpreting the results, the individual included in the exclusion criteria was set as an individual who moves around a specific area without exploration through swimming.

As shown in FIGS. 14 to 16, when the GTC310-01 antibody according to the present invention was administered, the number of times it crossed the target increased significantly, and the time taken to reach the platform significantly decreased.

Through these experiments, it was found that the antibody according to the present invention had a prophylactic, ameliorating, or therapeutic effect on brain and nervous system diseases.

**[Example 7] Evaluation of therapeutic effect of antibodies according to the present invention for Alzheimer's - 6xTg mice**

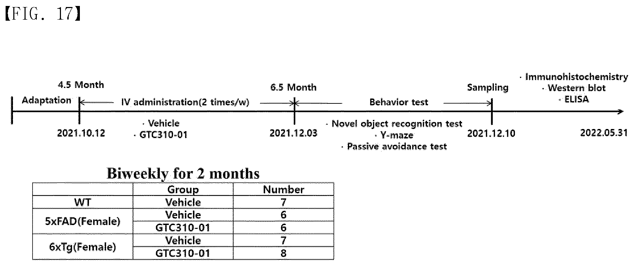

In order to evaluate the therapeutic ability of the antibody according to the present invention against Alzheimer's disease, groups were designed as shown in FIG. 17. Specifically, female 5xFAD mice with induced Alzheimer's disease and female 6xTg mice with induced Alzheimer's disease that underwent an adaptation period of 4.5 months were intravenously injected with GTC310-01 mouse antibody of Preparation Example 1 at an amount of 10mpk for 2 months. However, glatiramer acetate (GA, Copaxone®) was intravenously injected at an amount of 100 $\mu$g for 2 months as a positive control. After 2 months, the Y-maze test, novel object recognition experiment, and passive avoidance test were conducted.

**1. Characteristics of 6xTg mice**

Although the 5xFAD mice is widely used as an Alzheimer's-induced model, thee 6xTg mice used in the following experiment is a mouse model in which not only amyloid-$\beta$ (A$\beta$42) but also tau protein (MAPT) is mutated and accumulated, and thus is a model that is closer to a human disease model than the 5xFAD mouse, thus making it an ideal animal model for neurological diseases including Alzheimer's.

**2. Y-maze test**

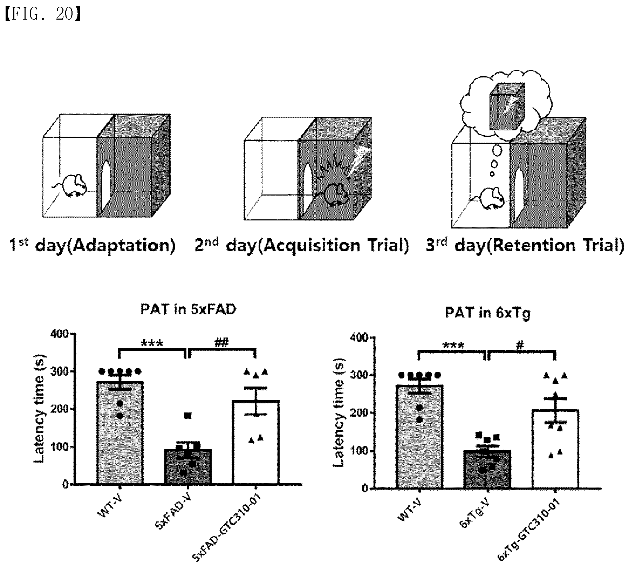

For the Y-maze test, a Y-shaped maze was used which had three identical arms having a length of 40 cm (with a 15-cm high wall) at an angle of 120 degrees from each other. This experiment was a behavioral experiment using the rodent's instinctive exploring habit and was based on the fact that there is a high possibility of exploring a new area. The higher the degree that the test animal remembered the arm it had just explored and tried not to enter the same arm, the higher the animal's memory level. An exploring time of 8 minutes was given to each individual, and the final results were expressed as spontaneous alteration (%) values in FIG. 20. The spontaneous alteration (%) value was calculated by Expression 1. Here, behavioral patterns were analyzed using SMART VIDEO TRACKING Software (Panlab, USA).

[Equation 1]

$$\text{Spontaneous alteration (\%)} = \frac{number\ of\ triplet}{total\ arm\ entry - 2}$$

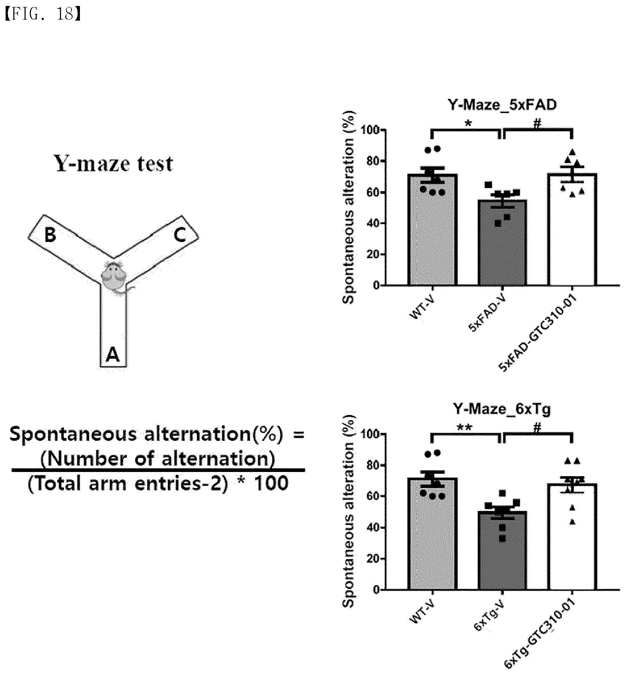

As shown in FIG. 18, in this experiment for measuring the relative frequency at which the test animal perceives the surrounding clues and sequentially enters the maze, it was found that in the case where the mice with induced Alzheimer's disease were administered with the GTC310-01 antibody according to the present invention, the mice had a spontaneous alteration value at a level similar to that of a normal control. Furthermore, mice of the 6xTg model, which is more similar to the human disease model, had a spontaneous alteration value more similar to that of the normal control group than the 5xFAD mouse.

**3. Novel Object Recognition**

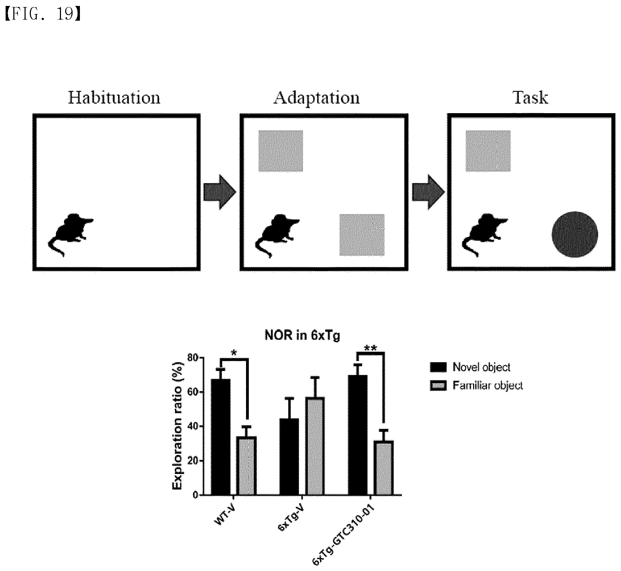

The novel object recognition test was performed to evaluate memory using two different objects in a 40 cm $\times$ 40 cm acrylic cage. After causing the test animal to acclimatize to the inside of the acrylic cage, two objects were placed at certain locations and the animal was allowed to perceive the objects freely. Then, the time for exploring each object was measured. 24-hour delay was given to each individual, and only one object was changed to another at the same location. Here, in a case where the animal perceives the changed object as a novel object and spends a longer exploring time, it may be determined that the animal has better memory. In a case where the animal does not remember the objects that it explored 24 hours before, the animal fails to distinguish between the novel object and the old object, and thus there is a possibility that the animal explores equally both objects. The animal was allowed to explore freely for a total of 10 minutes, and the result was expressed as a preference index (which equals novel object exploring time/total exploring time) in FIG. 19. Here, the analysis was performed using SMART VIDEO TRACKING Software (Panlab, USA).

As shown in FIG. 19, the preference index for a new object was lower in the mice with induced Alzheimer's disease compared with the normal control. However, when the GTC310-01 antibody according to the present invention was administered, the preference value was higher than that of the normal control in the 6xTg model, which is closer to the human disease model.

**4. Passive Avoidance Test**

The passive avoidance test experimental equipment is divided into two zones, a bright chamber with lighting and a dark chamber, and the floor is made of wire mesh. On the first day, the mice were allowed to freely move and adapted. The following day, each of the 5xFAD mice and 6xTg mice were adapted in a lighted chamber without the lights on for 1 minutes, then the lights were turned on and the mice were adapted for 2 minutes, and as soon as the mice were moved to the dark chamber, an electric shock was applied at 0.5mA for 1 second to conduct a learning test. A teat trial was conducted for each mouse on the day following the learning test. 5xFAD mice and 6xTg mice were placed in the chamber with lights on, and the time taken for all four paws of the 5xFAD mice and 6xTg mice to enter (latency time) was specified to within 300 seconds. As a result, as shown in FIG. 20, when the GTC310-01 antibody was administered to 5xFAD mice as well as 6xTg mice, the recovery was close to that of the normal control group.

Although the present invention has been described in detail above, the scope of the present invention is not limited thereto. It will be obvious to those skilled in the art that various modifications and changes can be made without departing from the technical spirit of the present invention described in the claims.

Industrial Applicability

The present invention relates to a novel binding molecule that is excellent for preventing, ameliorating or treating a brain and nervous system disease, and uses thereof to prevent or treat brain and nervous system diseases, particularly neurodegenerative diseases or neuroinflammatory diseases.

**Claims**

1. A binding molecule comprising:

   a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

2. The binding molecule according to claim 1,
   wherein the binding molecule comprises:

   a heavy chain variable region comprising a heavy chain CDR1 encoded by the nucleotide sequence represented by SEQ ID NO: 13, a heavy chain CDR2 encoded by the nucleotide sequence represented by SEQ ID NO: 14, and a heavy chain CDR3 encoded by the nucleotide sequence represented by SEQ ID NO: 15; and a light chain variable region comprising a light chain CDR1 encoded by the nucleotide sequence represented by SEQ ID NO: 16, a light chain CDR2 encoded by the nucleotide sequence represented by SEQ ID NO: 17, and a light chain CDR3 encoded by the nucleotide sequence represented by SEQ ID NO: 18.

3. The binding molecule according to claim 1,
   wherein the binding molecule comprises:

   a heavy chain variable region consisting of the amino acid sequence represented by SEQ ID NO: 11; and a light chain variable region consisting of the amino acid sequence represented by SEQ ID NO: 12.

4. The binding molecule according to claim 1,
   wherein the binding molecule comprises:

   a heavy chain variable region encoded by the nucleotide sequence represented by SEQ ID NO: 19; and a light chain variable region encoded by the nucleotide sequence represented by SEQ ID NO: 20.

**5.** The binding molecule according to claim 1,
wherein the binding molecule further comprises a fragment crystallization region (Fc region) or a constant region.

**6.** The binding molecule according to claim 5,
wherein the Fc region is the Fc region of an IgA, IgD, IgE, IgM, IgG1, IgG2, IgG3, or IgG4 antibody, or a hybrid Fc region.

**7.** The binding molecule according to claim 5,
wherein the binding molecule further comprises a heavy chain constant region consisting of an amino acid sequence selected from the group consisting of SEQ ID NOs: 21, 23, 24, 27, 28, 29, 30 and 32.

**8.** The binding molecule according to claim 5,
wherein the binding molecule further comprises a light chain constant region consisting of an amino acid sequence selected from the group consisting of SEQ ID NOs: 22, 25, 26, and 31.

**9.** The binding molecule according to claim 5,
wherein the binding molecule further comprises:

a heavy chain constant region encoded by the nucleotide sequence represented by SEQ ID NO: 33 or 35; and
a light chain constant region encoded by the nucleotide sequence represented by SEQ ID NO: 34 or 36.

**10.** The binding molecule according to claim 1,
wherein the binding molecule comprises:

a heavy chain consisting of the amino acid sequence represented by SEQ ID NO: 37 or 38; and
a light chain consisting of the amino acid sequence represented by SEQ ID NO: 39 or 40.

**11.** The binding molecule according to claim 10,
wherein the binding molecule comprises:

a heavy chain consisting of the amino acid sequence represented by SEQ ID NO: 37; and
a light chain consisting of the amino acid sequence represented by SEQ ID NO: 39.

**12.** The binding molecule according to claim 10,
wherein the binding molecule comprises:

a heavy chain consisting of the amino acid sequence represented by SEQ ID NO: 38; and
a light chain consisting of the amino acid sequence represented by SEQ ID NO: 40.

**13.** The binding molecule according to claim 10,
wherein the binding molecule comprises:

a heavy chain encoded by the nucleotide sequence represented by SEQ ID NO: 41 or 43; and
a light chain encoded by the nucleotide sequence represented by SEQ ID NO: 42 or 44.

**14.** The binding molecule according to claim 1,
wherein the binding molecule comprises:

a heavy chain encoded by the nucleotide sequence represented by SEQ ID NO: 41; and
a light chain encoded by the nucleotide sequence represented by SEQ ID NO: 42.

**15.** The binding molecule according to claim 1,
wherein the binding molecule comprises:

a heavy chain encoded by the nucleotide sequence represented by SEQ ID NO: 43; and
a light chain encoded by the nucleotide sequence represented by SEQ ID NO: 44.

**16.** The binding molecule according to claim 1,

wherein the binding molecule is an antibody or a fragment thereof.

17. The binding molecule according to claim 16,
wherein the antibody is a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof.

18. A nucleic acid molecule encoding the binding molecule of any one of claims 1 to 17.

19. An expression vector into which the nucleic acid molecule of claim 18 is inserted.

20. A host cell line transfected with the expression vector of claim 19.

21. An antibody-drug conjugate (ADC) comprising an antibody and a drug
wherein the antibody comprising:

a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and
a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

22. A pharmaceutical composition for preventing or treating a brain and nervous system disease that comprises, as an active ingredient, any one of the binding molecules of claims 1 to 17 or claim 21.

23. The pharmaceutical composition according to claim 22,
wherein the brain and nervous system disease is a neurodegenerative or neuroinflammatory disease.

24. The pharmaceutical composition according to claim 23,
wherein the neurodegenerative or neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, cortical degeneration syndrome, multiple system atrophy, progressive supranuclear palsy, nervous system autoimmune disease, spinocerebellar ataxia, inflammatory and neuropathic pain, cerebrovascular disease, spinal cord injury, and tauopathy.

25. A chimeric antigen receptor (CAR) comprising an antigen-specific binding domain, a linking domain and a CD3 zeta (ζ) signaling domain,
wherein the antigen-specific binding domain is:

a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and
a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

26. A pharmaceutical composition for preventing or treating a brain and nervous system disease comprising the chimeric antigen receptor of claim 25 as an active ingredient.

27. A diagnostic composition for a brain and nervous system disease comprising the binding molecule of claim 1.

28. A diagnostic kit for a brain and nervous system disease comprising the composition of claim 27.

29. A method for providing information on diagnosis of a brain and nervous system disease comprising a step of measuring the expression level of Lrig-1 protein present on the surface of T cells in a biological sample isolated from a target subject using the binding molecule of claim 1.

**30.** A method for preventing or treating a brain and nervous system disease **characterized by** a binding molecule comprising:

a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and
a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

**31.** A method for preventing or treating a brain and nervous system disease according to claim 30,

wherein the binding molecule is an antibody or a fragment thereof,
wherein the antibody is a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof.

**32.** A method for preventing or treating a brain and nervous system disease by administrating an antibody-drug conjugate (ADC) comprising an antibody and a drug:
wherein the antibody comprising:

a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and
a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

**33.** A method for preventing or treating a brain and nervous system disease **characterized by**:

a chimeric antigen receptor (CAR) comprising an antigen-specific binding domain, a linking domain and a CD3 zeta ($\zeta$) signaling domain,
wherein the antigen-specific binding domain is:

a heavy chain variable region comprising a heavy chain CDR1 consisting of the amino acid sequence represented by SEQ ID NO: 5, a heavy chain CDR2 consisting of the amino acid sequence represented by SEQ ID NO: 6, and a heavy chain CDR3 consisting of the amino acid sequence represented by SEQ ID NO: 7; and
a light chain variable region comprising a light chain CDR1 consisting of the amino acid sequence represented by SEQ ID NO: 8, a light chain CDR2 consisting of the amino acid sequence represented by SEQ ID NO: 9, and a light chain CDR3 consisting of the amino acid sequence represented by SEQ ID NO: 10.

【FIG. 1】

Leucine Rich Region (LRR) Domain

【FIG. 2】

3 Immunoglobulin-like domain

【FIG. 3】

| Gene | Fold change | Reference |
|---|---|---|
| GPR83 | 29.5 | J. Immunol. 2006 |
| Nrp1 | 23.7 | Eur. J. Immunol. 2004 |
| Lag3 | 3.1 | Immunity. 2003 |
| Ikzf4 | 4.2 | Science. 2009 |

【FIG. 4】

【FIG. 5】

【FIG. 6】

【FIG. 7】

【FIG. 8】

【FIG. 9】

| | Mice strain | Mice No. | Test article | Dosing regimen | | | Note |
|---|---|---|---|---|---|---|---|
| | | | | Dose | Frequency | Route | |
| G1 | WT | 12 | Vehicle | - | - | | WT Ctrl |
| G2 | | 10 | Vehicle | - | - | | Tg Ctrl |
| G3 | 5xFAD | 8 | Glatiramer acetate | 100 ug | BIW+QW x 3 wks | SC | Immune modulator (random polymer composed of four AA of MBP) |
| G4 | | 10 | GTC110-04 | 10 mpk | BIW x 4 wks | IV | anti-Lrig1 antibody |
| G5 | | 10 | GTC310-01 | 10 mpk | BIW x 3 wks | IV | anti-Lrig1 antibody |

【FIG. 10】

5×FAD Tg     GA     GTC110-04     GTC310-01

【FIG. 11】

A: 5xFAD Tg
B: +GA
C: GTC110-04
D: GTC310-01

【FIG. 12】

A: WT
B: 5xFAD Tg
C: +GA
D: GTC110-04
E: GTC310-01

【FIG. 13】

NOR

A: WT
B: 5xFAD Tg
C: +GA
D: GTC110-04
E: GTC310-01

【FIG. 14】

A: WT
B: 5xFAD Tg
C: +GA
D: GTC110-04
E: GTC310-01

【FIG. 15】

A: WT
B: 5xFAD Tg
C: +GA
D: GTC110-04
E: GTC310-01

【FIG. 16】

MWM-Acquisition test

【FIG. 17】

| | Group | Number |
|---|---|---|
| WT | Vehicle | 7 |
| 5xFAD(Female) | Vehicle | 6 |
| | GTC310-01 | 6 |
| 6xTg(Female) | Vehicle | 7 |
| | GTC310-01 | 8 |

【FIG. 18】

Y-maze test

$$\text{Spontaneous alternation(\%)} = \frac{\text{(Number of alternation)}}{\text{(Total arm entries-2)}} * 100$$

【FIG. 19】

Habituation       Adaptation       Task

NOR in 6xTg

【FIG. 20】

1st day(Adaptation)    2nd day(Acquisition Trial)    3rd day(Retention Trial)

PAT in 5xFAD

PAT in 6xTg

## INTERNATIONAL SEARCH REPORT

| | International application No. |
|---|---|
| | **PCT/KR2022/001498** |

### A. CLASSIFICATION OF SUBJECT MATTER

**C07K 16/28**(2006.01)i; **A61K 47/68**(2017.01)i; **A61P 25/00**(2006.01)i; **A61P 25/28**(2006.01)i; **A61K 39/00**(2006.01)i

According to International Patent Classification (IPC) or to both national classification and IPC

### B. FIELDS SEARCHED

Minimum documentation searched (classification system followed by classification symbols)

C07K 16/28(2006.01); A61K 47/68(2017.01); C12Q 1/6883(2018.01); C12Q 1/6886(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: Lrig-1, 항체(antibody), 중쇄(heavy chain), 경쇄(light chain), 뇌신경계 질환, 예방 (prevention), 치료(treatment), 제약(drug), 진단(diagnosis), 키트(kit), 항체-약물 결합체(antibody-drug conjugate, ADC), 키메 라 항원 수용체(chimera antigen receptor, CAR)

### C. DOCUMENTS CONSIDERED TO BE RELEVANT

| Category* | Citation of document, with indication, where appropriate, of the relevant passages | Relevant to claim No. |
|---|---|---|
| Y | KR 10-2018-0117067 A (GOOD T CELLS, INC.) 26 October 2018 (2018-10-26)<br>See abstract, claims 1, 8-11 and 16-21, and SEQ ID NOs: 21, 23, 30 and 37-40. | 1,5-8,16-29 |
| A | | 2-4,9-15 |
| Y | NCBI. Genbank accession no. CAG17619.1 (26 July 2016).<br>See entire document. | 1,5-8,16-29 |
| Y | KR 10-2020-0112745 A (GOOD T CELLS, INC.) 05 October 2020 (2020-10-05)<br>See claims 1, 19-20 and 23-25. | 22-24,26-29 |
| A | KR 10-2020-0043301 A (GOOD T CELLS, INC.) 27 April 2020 (2020-04-27)<br>See entire document. | 1-29 |
| A | KR 10-2020-0011377 A (GOOD T CELLS, INC.) 03 February 2020 (2020-02-03)<br>See entire document. | 1-29 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 May 2022** | **09 May 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2022/001498** |

---

**Box No. I**       **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13ter.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13ter.1(a)).

      ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.

**PCT/KR2022/001498**

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **30-33**
   because they relate to subject matter not required to be searched by this Authority, namely:

   Claims 30-33 pertain to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

International application No.

**PCT/KR2022/001498**

| Patent document cited in search report | | | Publication date (day/month/year) | Patent family member(s) | | | Publication date (day/month/year) |
|---|---|---|---|---|---|---|---|
| KR | 10-2018-0117067 | A | 26 October 2018 | CN | 110799534 | A | 14 February 2020 |
| | | | | CN | 110945024 | A | 31 March 2020 |
| | | | | EP | 3613771 | A1 | 26 February 2020 |
| | | | | EP | 3613772 | A2 | 26 February 2020 |
| | | | | JP | 2020-517241 | A | 18 June 2020 |
| | | | | JP | 2020-517248 | A | 18 June 2020 |
| | | | | KR | 10-2018-0117066 | A | 26 October 2018 |
| | | | | KR | 10-2086649 | B1 | 23 April 2020 |
| | | | | US | 2020-0048343 | A1 | 13 February 2020 |
| | | | | US | 2020-0131261 | A1 | 30 April 2020 |
| | | | | WO | 2018-194380 | A2 | 25 October 2018 |
| | | | | WO | 2018-194380 | A3 | 11 April 2019 |
| | | | | WO | 2018-194381 | A1 | 25 October 2018 |
| KR | 10-2020-0112745 | A | 05 October 2020 | CN | 113811330 | A | 17 December 2021 |
| | | | | EP | 3943107 | A1 | 26 January 2022 |
| | | | | WO | 2020-190104 | A1 | 24 September 2020 |
| KR | 10-2020-0043301 | A | 27 April 2020 | CN | 112912402 | A | 04 June 2021 |
| | | | | CN | 113056485 | A | 29 June 2021 |
| | | | | EP | 3868782 | A1 | 25 August 2021 |
| | | | | EP | 3882266 | A1 | 22 September 2021 |
| | | | | KR | 10-2020-0043302 | A | 27 April 2020 |
| | | | | KR | 10-2302393 | B1 | 16 September 2021 |
| | | | | KR | 10-2306346 | B1 | 30 September 2021 |
| | | | | WO | 2020-080853 | A1 | 23 April 2020 |
| | | | | WO | 2020-080854 | A1 | 23 April 2020 |
| KR | 10-2020-0011377 | A | 03 February 2020 | CN | 112543644 | A | 23 March 2021 |
| | | | | EP | 3827837 | A1 | 02 June 2021 |
| | | | | JP | 2021-532119 | A | 25 November 2021 |
| | | | | KR | 10-2263643 | B1 | 10 June 2021 |
| | | | | US | 2021-0347849 | A1 | 11 November 2021 |
| | | | | WO | 2020-022782 | A1 | 30 January 2020 |

Form PCT/ISA/210 (patent family annex) (July 2019)

**REFERENCES CITED IN THE DESCRIPTION**

**Patent documents cited in the description**

- US 4554101 A